# EUROPEAN PATENT APPLICATION

(11) **EP 4 062 934 A1**
(43) Date of publication of application: **28.09.2022**
(21) Application number: 20891244.4
(22) Date of filing: 18.11.2020
(51) Int. Cl.: A61K 39/395, A61K 31/713, A61K 45/06, A61P 35/00, C07K 16/28

(54) **COMPOSITION FOR PREVENTING OR TREATING CANCER, COMPRISING ANTI-CD300C MONOCLONAL ANTIBODIES**

(30) Priority: 18.11.2019 KR 20190147496; 17.11.2020 KR 20200154076
(71) Applicant: CentricsBio, Inc., Seoul 05836 (KR)
(72) Inventor: JEON, Jaewon, Gyeonggi-do 16512 (KR); LEE, Suin, Guri-si, Gyeonggi-do 11949 (KR); KIM, Woochang, Chuncheon-si, Gangwon-do 24434 (KR); KIM, Haneul, Namyangju-si, Gyeonggi-do 12226 (KR)
(74) Representative: Dehns
(86) International application number: PCT/KR2020/016264
(87) International publication number: WO 2021/101244

(57) **Abstract**

The present disclosure relates to an anti-CD300c monoclonal antibody, a composition for preventing or treating cancer which comprises the same, a composition for anticancer immunotherapy which comprises the same, and the like. The anti-CD300c monoclonal antibody according to the present disclosure binds to a CD300c antigen with high specificity and also promotes anticancer immune responses. Thus, this anti-CD300c monoclonal antibody is expected to be effectively used against growth, development, metastasis, and the like of various cancers.

## Description

### Technical Field

The present disclosure relates to an anti-CD300c monoclonal antibody, a composition for preventing or treating cancer which comprises the antibody, a composition for anticancer immunotherapy which comprises the antibody, and the like.

### Background Art

Cancer is one of the diseases that account for the largest share of the causes of death in modern people. This disease is caused by changes in normal cells due to genetic mutations that result from various causes and refers to a malignant tumor that does not follow differentiation, proliferation, growth pattern, or the like of normal cells. Cancer is characterized by "uncontrolled cell growth." This abnormal cell growth causes formation of a mass of cells called a tumor, which infiltrates the surrounding tissues and, in severe cases, may metastasize to other organs of the body. Cancer is an intractable chronic disease that is not fundamentally cured in many cases even if it is treated with surgery, radiotherapy, chemotherapy, and the like, causes pain to patients, and ultimately leads to death. In particular, in recent years, the global cancer incidence rate is increasing by 5% or higher every year due to increased elderly population, environmental deterioration, or the like. According to the WHO report, it is estimated that within the next 25 years the number of cancer patients will increase to 30 million, of which 20 million will die from cancer.

Cancer drug treatments, that is, cancer chemotherapies are generally cytotoxic compounds, and treat cancer by attacking and killing cancer cells. However, these chemotherapies exhibit high adverse effects since they damage not only cancer cells but also normal cells. Thus, targeted cancer chemotherapies have been developed to decrease adverse effects. These targeted cancer chemotherapies were able to exhibit decreased adverse effects, but had a limitation in that resistance occurs with a high probability. Therefore, in recent years, interest in cancer immunotherapies, which use the body's immune system to decrease problems due to toxicity and resistance, is rapidly increasing. As an example of such cancer immunotherapies, immune checkpoint inhibitors have been developed which specifically bind to PD-L1 on the surface of cancer cells and inhibit its binding to PD-1 on T cells so that T cells are activated and attack cancer cells (Korean Patent Laid-Open Publication No. 10-2018-0099557). However, even these immune checkpoint inhibitors are not effective in various types of cancer. Therefore, there is a need to develop novel cancer immune therapeutics that exhibit an equivalent therapeutic effect in various cancers.

### Disclosure of Invention

### Technical Problem

The present disclosure has been made to solve the problems of the prior art as described above. An object of the present disclosure is to provide an anti-CD300c monoclonal antibody, a composition for preventing or treating cancer which comprises the antibody, a composition for anticancer immunotherapy comprising the antibody, and the like.

However, the technical problem to be solved by the present disclosure is not limited to the above-mentioned problems, and other problems which are not mentioned will be clearly understood by those skilled in the art from the following description.

### Solution to Problem

According the present disclosure, there is provided an anti-CD300c monoclonal antibody, comprising any one or more complementarity-determining region (CDR) sequences selected from the group consisting of SEQ ID NOs: 2, 4, 6, 8, 10, 12, 14, 16, 18, 20, 22, 24, 26, 28, 30, 32, 34, 36, 38, 40, 42, 44, 46, 48, and 50. The CDR sequences may include amino acid sequences having 90% or higher, more preferably 95% or higher, and most preferably 98% or higher sequence homology to any one or more CDR sequences selected from the group consisting of SEQ ID NOs: 2, 4, 6, 8, 10, 12, 14, 16, 18, 20, 22, 24, 26, 28, 30, 32, 34, 36, 38, 40, 42, 44, 46, 48, and 50. The "% sequence homology" with respect to amino acid sequences is determined by comparing two optimally aligned sequences over a comparison window, wherein the portion of the amino acid sequence in the comparison window may include additions or deletions (that is, gaps) as compared to the reference sequence (that does not include additions or deletions) for optimal alignment of the two sequences.

In an embodiment of the present disclosure, the anti-CD300c monoclonal antibody may have inter-species cross-reactivity. The inter-species cross-reactivity may preferably mean cross-reactivity between a human-derived CD300c antigen and a mammal-derived CD300c antigen, and more preferably cross-reactivity between a human antigen and a mouse antigen.

In addition, according to the present disclosure, there is provided a pharmaceutical composition for preventing or treating cancer, comprising the anti-CD300c monoclonal antibody as an active ingredient.

In an embodiment of the present disclosure, the cancer may preferably be colorectal cancer, rectal cancer, colon cancer, thyroid cancer, oral cancer, pharyngeal cancer, laryngeal cancer, cervical cancer, brain cancer, lung cancer, ovarian cancer, bladder cancer, kidney cancer, liver cancer, pancreatic cancer, prostate cancer, skin cancer, tongue cancer, breast cancer, uterine cancer, stomach cancer, bone cancer, blood cancer, or the like. However, the cancer is not limited thereto and may include any type of cancer in which the CD300c protein is expressed on the surface of cancer cells.

In another embodiment of the present disclosure, the pharmaceutical composition may further comprise other conventional cancer immunotherapies or chemotherapies. The immunotherapy may preferably be, but is not limited to, anti-PD-1, anti-PD-Ll, anti- CTLA-4, anti-KIR, anti-LAG3, anti-CD 137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, anti-TIGIT, or the like, and may include any substance as long as it is currently used as an immunotherapy. In addition, the chemotherapy may preferably be, but is not limited to, doxorubicin, cisplatin, gemcitabine, oxaliplatin, 5-FU, cetuximab, panitumumab, nimotuzumab, necitumumab, a cancer antigen, an anticancer virus, or the like, and may include any substance as long as it is currently used as a chemotherapy. The cancer antigen is a cancer vaccine specific to carcinoma and may preferably be NY-ESO-1 as a bladder cancer-specific cancer antigen, HER2 as a breast cancer-specific cancer antigen, CEA as a colorectal cancer-specific cancer antigen, and VEGFR1 or VEGFR2 as a lung cancer-specific cancer antigen. However, the cancer antigen is not limited thereto and may include any type of cancer antigen as long as it is known as a cancer vaccine. Examples of the anticancer virus include Imlygic and Pexa-Vec. However, the anticancer virus is not limited thereto and may include any anticancer virus as long as it is known as an anticancer virus. In a case where the cancer therapy is further included, such a therapy may preferably be co-administered with the monoclonal antibody of the present disclosure, may be in a form of being bound to the monoclonal antibody of the present disclosure, or may be included together with the monoclonal antibody of the present disclosure in a vehicle.

In yet another embodiment of the present disclosure, the pharmaceutical composition is characterized in that it inhibits proliferation, survival, metastasis, recurrence, therapy resistance, or the like of cancer or cancer stem cells. However, the effect is not limited thereto and may include any effect exerted by the pharmaceutical composition of the present disclosure.

In addition, according to the present disclosure, there is provided a cancer immunotherapy, comprising the anti-CD300c monoclonal antibody as an active ingredient.

In addition, according to the present disclosure, there is provided an adjuvant for anticancer therapy, comprising the anti-CD300c monoclonal antibody as an active ingredient.

In an embodiment of the present disclosure, the adjuvant may activate an immune function of immune cells to result in enhanced anticancer therapeutic effects.

In another embodiment of the present disclosure, the anticancer therapy may be radiation therapy, chemotherapy, immunotherapy, or the like.

In addition, according to the present disclosure, there is provided a method for treating cancer, comprising a step of administering to an individual a composition comprising the anti-CD300c monoclonal antibody as an active ingredient.

In addition, according to the present disclosure, there is provided a use of a composition, which comprises the anti-CD300c monoclonal antibody as an active ingredient, for preventing or treating cancer.

In addition, according to the present disclosure, there is provided a use of the anti-CD300c monoclonal antibody for the manufacture of a medicament for use in cancer treatment.

### Advantageous Effects of Invention

The anti-CD300c monoclonal antibody according to the present disclosure specifically binds, with high binding affinity, to CD300c expressed on the surface of various cancers, which activates T cells and at the same time promotes differentiation into M1 macrophages so that proliferation of cancer cells can be effectively inhibited. Thus, the anti-CD300c monoclonal antibody can be effectively used as an immunotherapy for various cancers. In addition, the anti-CD300c monoclonal antibody according to the present disclosure can exhibit a further increased therapeutic effect through co-administration with a conventional cancer immunotherapy, and also has inter-species cross-reactivity that allows the antibody to be widely applied to various mammals. In addition, it is expected that in a case where resistant cancer cells showing the ability to resist apoptosis are treated with the anti-CD300c monoclonal antibody of the present disclosure, this antibody can remarkably weaken resistance of the cancer cells, thereby showing excellent efficacy in preventing cancer recurrence. In addition, in general, cancer cells inhibit production of the proinflammatory cytokine IL-2 to evade the immune system. It was identified that the anti-CD300c monoclonal antibody activates the immune system by restoring production of IL-2 blocked by these cancer cells, which induces cancer cell death. Thus, it is believed that the anti-CD300c monoclonal antibody can be utilized as a more fundamental cancer immunotherapy.

### Brief Description of Drawings

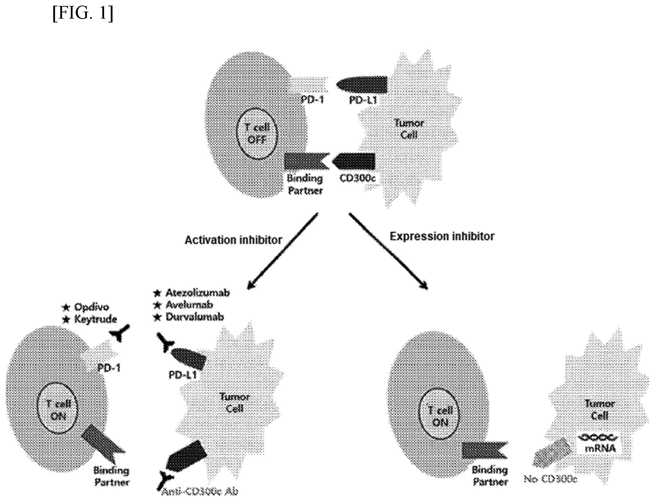

FIG. 1 illustrates a schematic diagram, briefly showing the mechanism by which the anti-CD300c monoclonal antibody and/or CD300c siRNA of the present disclosure exhibits an anticancer effect.

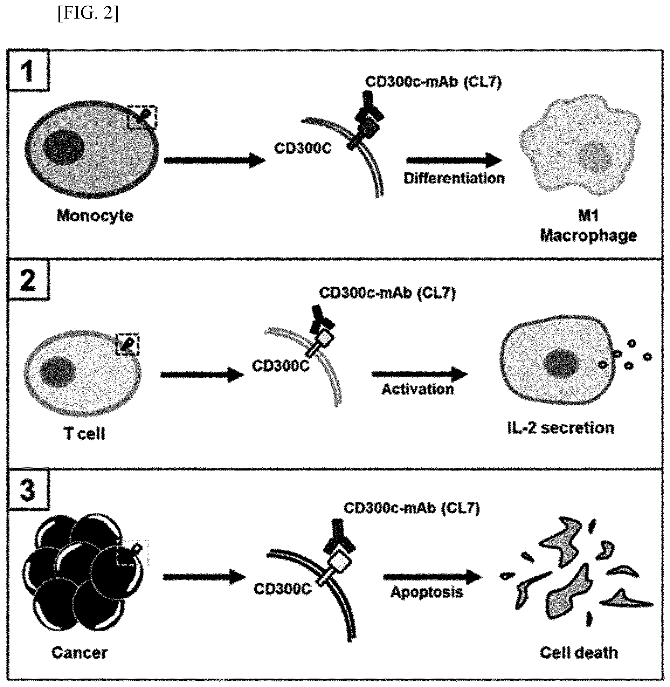

FIG. 2 illustrates a schematic diagram, briefly showing the mechanism by which the anti-CD300c monoclonal antibody of the present disclosure acts on monocytes, T cells, and cancer cells, respectively.
FIG. 3 illustrates results obtained by performing SDS-PAGE on the anti-CD300c monoclonal antibodies according to an embodiment of the present disclosure under a non-reducing condition.
FIG. 4 illustrates results obtained by performing SDS-PAGE on the anti-CD300c monoclonal antibodies according to an embodiment of the present disclosure under a reducing condition.
FIG. 5 illustrates results obtained by identifying the binding affinity, to a CD300c antigen, of the anti-CD300c monoclonal antibody according to an embodiment of the present disclosure.
FIG. 6 illustrates results obtained by identifying, with ELISA, the T cell activation ability of the anti-CD300c monoclonal antibody according to an embodiment of the present disclosure.
FIG. 7 illustrates results obtained by identifying, with ELISA, the differentiation capacity into M1 macrophages of the anti-CD300c monoclonal antibodies according to an embodiment of the present disclosure.
FIG. 8 illustrates results obtained by identifying, with ELISA, the capacity of the anti-CD300c monoclonal antibodies according to an embodiment of the present disclosure for causing differentiation into M1 macrophages.
FIG. 9 illustrates results obtained by identifying, with ELISA, the differentiation capacity into M1 macrophages depending on concentrations of the anti-CD300c monoclonal antibodies according to an embodiment of the present disclosure.
FIG. 10 illustrates results obtained by identifying, with ELISA, the differentiation capacity into M1 macrophages depending on concentrations of the anti-CD300c monoclonal antibody according to an embodiment of the present disclosure.
FIG. 11 illustrates results obtained by identifying, through cell shape, the differentiation capacity into M1 macrophages of the anti-CD300c monoclonal antibody according to an embodiment of the present disclosure.
FIG. 12 illustrates results obtained by identifying, with ELISA, the differentiation capacity into M1 macrophages of the anti-CD300c monoclonal antibody CL7 according to an embodiment of the present disclosure.
FIG. 13 illustrates results obtained by comparing, with ELISA, the differentiation capacity into M1 macrophages between the anti-CD300c monoclonal antibodies according to an embodiment of the present disclosure and a cancer immunotherapy.
FIG. 14 illustrates results obtained by comparing, with ELISA, the differentiation capacity into M1 macrophages between the anti-CD300c monoclonal antibody CL7 according to an embodiment of the present disclosure and cancer immunotherapies.
FIG. 15 illustrates results obtained by comparing, with ELISA, the differentiation capacity into M1 macrophages between the anti-CD300c monoclonal antibody CL7 according to an embodiment of the present disclosure and cancer immunotherapies.
FIG. 16 illustrates results obtained by comparing, with ELISA, the differentiation capacity into M1 macrophages between the anti-CD300c monoclonal antibody CL7 according to an embodiment of the present disclosure and cancer immunotherapies.
FIG. 17 illustrates results obtained by comparing, with ELISA, the differentiation capacity from M0 macrophages into M1 macrophages between the anti-CD300c monoclonal antibody according to an embodiment of the present disclosure and a cancer immunotherapy.
FIG. 18 illustrates results obtained by comparing, with ELISA, the differentiation capacity into M1 macrophages between the anti-CD300c monoclonal antibody according to an embodiment of the present disclosure and a cancer immunotherapy.
FIG. 19 illustrates results obtained by identifying, with ELISA, the redifferentiation capacity from M2 macrophages into M1 macrophages of the anti-CD300c monoclonal antibody according to an embodiment of the present disclosure.
FIG. 20 illustrates results obtained by identifying, with ELISA, the redifferentiation capacity from M2 macrophages into M1 macrophages of the anti-CD300c monoclonal antibody according to an embodiment of the present disclosure.
FIG. 21 illustrates results obtained by identifying, with ELISA, the redifferentiation capacity from M2 macrophages into M1 macrophages of the anti-CD300c monoclonal antibody according to an embodiment of the present disclosure.
FIG. 22 illustrates results obtained by identifying, with ELISA, the redifferentiation capacity from M0, M1, and M2 macrophages into M1 macrophages of the anti-CD300c monoclonal antibody according to an embodiment of the present disclosure.
FIG. 23 illustrates results obtained by identifying, with differentiation capacity into M1 macrophages, the effects caused by co-administration of the anti-CD300c monoclonal antibody according to an embodiment of the present disclosure and an anti-PD-L1 immunotherapy.
FIG. 24 illustrates results obtained by identifying, with differentiation capacity into M1 macrophages, the effects caused by co-administration of the anti-CD300c monoclonal antibody according to an embodiment of the present disclosure and a cancer immunotherapy.
FIG. 25 illustrates results obtained by identifying the cancer cell growth inhibitory effects of the anti-CD300c monoclonal antibodies according to an embodiment of the present disclosure under a condition of 0% FBS.
FIG. 26 illustrates results obtained by identifying the cancer cell growth inhibitory effects of the anti-CD300c monoclonal antibodies according to an embodiment of the present disclosure under a condition of 0.1% FBS.
FIG. 27 illustrates results obtained by comparing the cancer cell (lung cancer) growth inhibitory effects of the anti-CD300c monoclonal antibodies according to an embodiment of the present disclosure and a cancer immunotherapy.
FIG. 28 illustrates results obtained by comparing the cancer cell (breast cancer) growth inhibitory effects of the anti-CD300c monoclonal antibodies according to an embodiment of the present disclosure and a cancer immunotherapy.
FIG. 29 illustrates results obtained by identifying the cancer cell growth inhibitory effects depending on concentrations of the anti-CD300c monoclonal antibody according to an embodiment of the present disclosure.
FIG. 30 illustrates results obtained by identifying the cancer cell growth inhibitory effects caused by co-administration of the anti-CD300c monoclonal antibody according to an embodiment of the present disclosure and a cancer immunotherapy.
FIG. 31 illustrates results obtained by identifying the cancer cell growth inhibitory effects caused by co-administration of the anti-CD300c monoclonal antibody according to an embodiment of the present disclosure and a cancer immunotherapy.
FIG. 32 illustrates results obtained by identifying the mechanism of action caused by co-administration of the anti-CD300c monoclonal antibody according to an embodiment of the present disclosure and a cancer immunotherapy.
FIG. 33 illustrates results obtained by identifying the binding specificity of the anti-CD300c monoclonal antibodies according to an embodiment of the present disclosure.
FIG. 34 illustrates results obtained by identifying the cross-reactivity, in mice, of the anti-CD300c monoclonal antibodies according to an embodiment of the present disclosure.
FIG. 35 illustrates results obtained by identifying the anti-cancer (colorectal cancer) effects, in mice, of the anti-CD300c monoclonal antibodies according to an embodiment of the present disclosure.
FIG. 36 schematically illustrates an experimental method for identifying the effects of the anti-CD300c monoclonal antibody according to an embodiment of the present disclosure on cancer growth *in vivo.*
FIG. 37 illustrates results obtained by identifying the cancer growth inhibitory effects *in vivo* of the anti-CD300c monoclonal antibody according to an embodiment of the present disclosure.
FIG. 38 illustrates results obtained by identifying the effects of the anti-CD300c monoclonal antibody according to an embodiment of the present disclosure on an increase in tumor-infiltrating lymphocytes under a tumor microenvironment *in vivo.* The scale bar indicates 50 um.
FIG. 39 illustrates results obtained by identifying the effects of the anti-CD300c monoclonal antibody according to an embodiment of the present disclosure on an increase in M1 macrophages *in vivo.*

### Best Mode for Carrying out Invention

The anti-CD300c monoclonal antibody of the present disclosure specifically binds, with high binding affinity, to a CD300c protein and effectively inhibits the mechanism of CD300c, which activates T cells and promotes differentiation into M1 macrophages so that growth of cancer cells, and development, metastasis, and the like of cancer can be effectively inhibited. Thus, the anti-CD300c monoclonal antibody can be effectively used for the treatment of various cancers that express a CD300c antigen on the surface.

As used herein, the term "antibody" refers to an immunoglobulin molecule that is immunologically reactive with a specific antigen, and includes all of polyclonal antibodies, monoclonal antibodies, and functional fragments thereof. In addition, the term may include forms produced by genetic engineering, such as chimeric antibodies (for example, humanized murine antibodies) and heterologous antibodies (for example, bispecific antibodies). Among these, the monoclonal antibodies are antibodies that exhibit single binding specificity and affinity against a single antigenic site (epitope). Unlike polyclonal antibodies including antibodies that exhibit specificity against different epitopes, the monoclonal antibodies exhibit binding specificity and affinity against a single epitope on an antigen, which allows for easy quality control as a therapeutic agent. In particular, the anti-CD300c monoclonal antibody of the present disclosure not only exhibits anticancer activity by itself by specifically binding to CD300c-expressing cancer cells, but also stimulates immune cells, thereby exhibiting maximized cancer cell-dependent anticancer activity. The antibody includes variable region(s) of a heavy chain and/or a light chain in terms of the constitution, wherein the variable region includes, as a primary structure thereof, a portion that forms an antigen-binding site of the antibody molecule. The antibody of the present disclosure may be composed of a partial fragment containing the variable region. Preferably, the variable region may be replaced by a soluble receptor for CD300c. However, the variable region is not limited thereto and may include anything as long as the thus formed antibody exhibits the same effect as the anti-CD300c monoclonal antibody of the present disclosure.

As used herein, the term "immunoglobulin" refers to a concept that encompasses both an antibody and an antibody-like molecule that has the same structural characteristics as an antibody and does not have antigenic specificity.

As used herein, the term "single-chain variable fragment (scFv)" refers to a protein in which light chain and heavy chain variable regions of an antibody are linked to each other via a linker consisting of a peptide sequence having about 15 amino acid residues. The scFv may be in an order of light chain variable domain-linker-heavy chain variable region, or an order of heavy chain variable region-linker-light chain variable region, and has the same or similar antigen specificity as its original antibody. The linking site is a hydrophilic flexible peptide chain mainly composed of glycine and serine. The 15-amino acid sequence of "(Gly-Gly-Gly-Gly-Ser)₃ or a sequence similar thereto is mainly used.

As used herein, the term "cancer immunotherapy" (also referred to as simply "immunotherapy") collectively refers to a cancer therapy or anticancer agent that activates an immune function of immune cells in the body to fight cancer cells. Examples thereof may include, but are not limited to, anti-PD-1, anti-PD-Ll, anti-CTLA-4, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, and anti-TIGIT.

As used herein, the term "adjuvant" refers to an auxiliary drug used for the purpose of assisting in the efficacy of a main drug, that is, a cancer therapy to improve and/or enhance its therapeutic effect, suppressing resistance to the main drug to improve and/or enhance its therapeutic effect, or preventing or alleviating harmful action of the main drug. The adjuvant of the present disclosure is not limited as long as it contains the anti-CD300c monoclonal antibody as an active ingredient.

As used herein, the term "prevention" means any action that inhibits or delays onset of diseases such as cancer by administration of the pharmaceutical composition according to the present disclosure.

As used herein, the term "treatment" means any action that ameliorates or beneficially alters symptoms of cancer or the like by administration of the pharmaceutical composition according to the present disclosure.

As used herein, the term "individual" refers to a subject to which the pharmaceutical composition of the present disclosure can be administered, and the subject is not limited.

As used herein, the term "pharmaceutical composition" may be characterized by being in the form of capsules, tablets, granules, injections, ointments, powders, or beverages, and the pharmaceutical composition may be characterized by being for application to humans. The pharmaceutical composition may be formulated in the form of oral preparations such as powders, granules, capsules, tablets, and aqueous suspensions, preparations for external use, suppositories, and sterile injectable solutions, respectively, according to conventional methods, and used. However, the pharmaceutical composition is not limited thereto. The pharmaceutical composition of the present disclosure may further comprise a pharmaceutically acceptable carrier. As the pharmaceutically acceptable carrier, a binder, a glidant, a disintegrant, an excipient, a solubilizer, a dispersant, a stabilizer, a suspending agent, a pigment, a flavor, and the like may be used for oral administration; a buffer, a preserving agent, a pain-relieving agent, a solubilizer, an isotonic agent, a stabilizer, and the like may be used in admixture for injections; and a base, an excipient, a lubricant, a preserving agent, and the like may be used for topical administration. The preparations of the pharmaceutical composition of the present disclosure may be prepared in various ways by being mixed with the pharmaceutically acceptable carrier as described above. For example, for oral administration, the pharmaceutical composition may be formulated in the form of tablets, troches, capsules, elixirs, suspensions, syrups, wafers, or the like. For injections, the pharmaceutical composition may be formulated in the form of unit dosage ampoules or multiple dosage forms. Alternatively, the pharmaceutical composition may be formulated into solutions, suspensions, tablets, capsules, sustained-release preparations, or the like.

Meanwhile, as examples of carriers, diluents, or excipients suitable for making preparations, lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinylpyrrolidone, water, methyl hydroxybenzoate, propyl hydroxybenzoate, talc, magnesium stearate, mineral oil, or the like may be used. In addition, a filler, an anti-coagulant, a lubricant, a wetting agent, a fragrance, an emulsifier, a preservative, and the like may further be included.

The route of administration of the pharmaceutical composition includes, but is not limited to, oral, intravenous, intramuscular, intraarterial, intramedullary, intradural, intracardiac, transdermal, subcutaneous, intraperitoneal, intranasal, intestinal, topical, sublingual, or rectal route. Oral or parenteral administration is preferred. As used herein, the term "parenteral" includes subcutaneous, intradermal, intravenous, intramuscular, intraarticular, intrabursal, intrasternal, intradural, intralesional, and intracranial injection or infusion techniques. The pharmaceutical composition of the present disclosure may also be administered in the form of suppositories for rectal administration.

The pharmaceutical composition of the present disclosure may vary widely depending on a variety of factors, including activity of a certain compound used, the patient's age, body weight, general health status, sex, diet, time of administration, route of administration, rate of excretion, drug combination, and severity of a certain disease to be prevented or treated. A dose of the pharmaceutical composition may vary depending on the patient's condition, body weight, severity of disease, drug form, route of administration, and duration, and may be appropriately selected by those skilled in the art. The pharmaceutical composition may be administered in an amount of 0.0001 to 500 mg/kg or 0.001 to 500 mg/kg, per day. Administration may be made once a day or several times a day. The dose is not intended to limit the scope of the invention in any way. The pharmaceutical composition according to the present disclosure may be formulated in the form of pills, sugar-coated tablets, capsules, liquids, gels, syrups, slurries, or suspensions.

Hereinafter, the following examples are provided to help the understanding of the present disclosure. However, the following examples are only provided for easier understanding of the present disclosure, and the scope of the present disclosure is not limited by the following examples.

### Examples

### Example 1: Selection of anti-CD300c monoclonal antibody

### 1.1. Construction of anti-CD300c monoclonal antibody library

In order to select anti-CD300c monoclonal antibodies, biopanning was performed using a lambda phage library, a kappa phage library, a VH3VL1 phage library, and an OPALTL phage library. More specifically, a CD300c antigen was added at a concentration of 5 µg/mL to an immunotube, and reaction was allowed to proceed for 1 hour so that the antigen was adsorbed on the surface of the immunotube. 3% skim milk was added to suppress non-specific reactions. Then, 10¹² PFU of the antibody phage library dispersed in 3% skim milk was added to each immunotube for antigen binding. Washing was performed 3 times using Tris buffered saline-Tween 20 (TBST) solution to remove non-specifically bound phages, and then single-chain variable fragment (scFv) phage antibodies, specifically bound to the CD300c antigen, were eluted using 100 mM triethylamine solution. The eluted phages were neutralized using 1.0 M Tris-HCl buffer (pH 7.8). Then, the resultant was subjected to E. coli ER2537 and infection was allowed to proceed at 37°C for 1 hour. The infected E. coli was applied onto LB agar medium containing carbenicillin. and cultured at 37°C for 16 hours. Then, the formed E. coli colonies were suspended using 3 mL of super broth (SB)-carbenicillin culture. Some of the suspension was stored at -80°C until use with the addition of 15% glycerol, and the remaining portion was reinoculated into SB-carbenicillin-2% glucose solution and cultured at 37°C. Then, the obtained culture was centrifuged, and biopanning was repeated 3 times again using the supernatant containing phage particles to obtain and concentrate antigen-specific antibodies.

After repeating the biopanning 3 times, E. coli containing the antibody gene was applied onto LB agar medium containing carbenicillin and cultured at 37°C for 16 hours. The formed E. coli colonies were inoculated again into SB-carbenicillin-2% glucose solution and cultured at 37°C until the absorbance (at OD 600 nm) reached 0.5. Then, IPTG was added and further cultured at 30°C for 16 hours. Thereafter, periplasmic extraction was performed. From the results, a library pool of antibodies, which specifically bind to the CD300c antigen, was primarily obtained.

### 1.2. Selection of anti-CD300c monoclonal antibody

In order to select anti-CD300c monoclonal antibodies that specifically bind, with high binding affinity, to a CD300c antigen, ELISA was performed using the library pool obtained in the same manner as in Example 1.1. More specifically, each of a CD300c antigen and a CD300a antigen in a coating buffer (0.1 M sodium carbonate, pH 9.0) was dispensed onto an ELISA plate at a concentration of 5 µg/mL per well, and then reaction was allowed to proceed at room temperature for 3 hours so that the antigen was bound to the plate. Washing was performed 3 times using phosphate buffered saline-Tween 20 (PBST) to remove unbound antigen, and then 350 µL of PBST supplemented with 2% bovine serum albumin (BSA) was added to each well. Reaction was allowed to proceed at room temperature for 1 hour, and washing was performed again using PBST. Then, 25 µg of periplasmic extract containing scFv obtained in the same manner as in Example 1.1 was added thereto, and reaction was allowed to proceed for 1 hour at room temperature for antigen binding. After 1 hour, washing was performed 3 times using PBST to remove unbound scFv, and then 4 µg/mL of an antibody for detection was added. Reaction was allowed to proceed again at room temperature for 1 hour. Subsequently, the unbound antibody for detection was removed using PBST. Then, anti-rabbit IgG to which HRP was bound was added and reaction was allowed to proceed at room temperature for 1 hour. The unbound antibody was removed again using PBST. TMB solution was added and reaction was allowed to proceed for 10 minutes for development. Then, 2 N sulfuric acid solution was added to terminate the development, and the absorbance was measured at 450 nm to identify the antibodies that specifically bind to the CD300c antigen.

### 1.3. Identification of anti-CD300c monoclonal antibody sequences

The nucleotide sequences of the anti-CD300c monoclonal antibodies, which were selected using the same method as in Example 1.2, were identified. More specifically, for each of the selected antibody clones, plasmid DNA was extracted therefrom using a plasmid miniprep kit. Then, DNA sequencing was performed to analyze complementarity-determining region (CDR) sequences. As a result, 25 types of anti-CD300c monoclonal antibodies having different amino acid sequences were obtained.

There were 3 types of anti-CD300c monoclonal antibodies selected using the lambda phage library: SL18 including the CDR sequence(s) of SEQ ID NO: 36 (the DNA sequence thereof is SEQ ID NO: 35), CL4 including the CDR sequence(s) of SEQ ID NO: 8 (the DNA sequence thereof is SEQ ID NO: 7), and CL5 including the CDR sequence(s) of SEQ ID NO: 10 (the DNA sequence thereof is SEQ ID NO: 9).

There were 10 types of anti-CD300c monoclonal antibodies selected using the kappa phage library: CK1 including the CDR sequence(s) of SEQ ID NO: 2 (the DNA sequence thereof is SEQ ID NO: 1), CK2 including the CDR sequence(s) of SEQ ID NO: 4 (the DNA sequence thereof is SEQ ID NO: 3), CK3 including the CDR sequence(s) of SEQ ID NO: 6 (the DNA sequence thereof is SEQ ID NO: 5), SK11 including the CDR sequence(s) of SEQ ID NO: 22 (the DNA sequence thereof is SEQ ID NO: 21), SK12 including the CDR sequence(s) of SEQ ID NO: 24 (the DNA sequence thereof is SEQ ID NO: 23), SK13 including the CDR sequence(s) of SEQ ID NO: 26 (the DNA sequence thereof is SEQ ID NO: 25), SK14 including the CDR sequence(s) of SEQ ID NO: 28 (the DNA sequence thereof is SEQ ID NO: 27), SK15 including the CDR sequence(s) of SEQ ID NO: 30 (the DNA sequence thereof is SEQ ID NO: 29), SK16 including the CDR sequence(s) of SEQ ID NO: 32 (the DNA sequence thereof is SEQ ID NO: 31), and SK17 including the CDR sequence(s) of SEQ ID NO: 34 (the DNA sequence thereof is SEQ ID NO: 33).

There were 10 types of anti-CD300c monoclonal antibodies selected using the VH3VL1 phage library: CB301_H3L1_A10 including the CDR sequence(s) of SEQ ID NO: 37 (the DNA sequence thereof is SEQ ID NO: 38), CB301_H3L1_A12 including the CDR sequence(s) of SEQ ID NO: 40 (the DNA sequence thereof is SEQ ID NO: 39), CL6 including the CDR sequence(s) of SEQ ID NO: 12 (the DNA sequence thereof is SEQ ID NO: 11), CB301_H3L1_E6 including the CDR sequence(s) of SEQ ID NO: 42 (the DNA sequence thereof is SEQ ID NO: 41), CL7 including the CDR sequence(s) of SEQ ID NO: 14 (the DNA sequence thereof is SEQ ID NO: 13), CB301_H3L1_F4 including the CDR sequence(s) of SEQ ID NO: 44 (the DNA sequence thereof is SEQ ID NO: 43), CL8 including the CDR sequence(s) of SEQ ID NO: 16 (the DNA sequence thereof is SEQ ID NO: 15), CB301_H3L1_G11 including the CDR sequence(s) of SEQ ID NO: 46 (the DNA sequence thereof is SEQ ID NO: 45), CL9 including the CDR sequence(s) of SEQ ID NO: 18 (the DNA sequence thereof is SEQ ID NO: 17), and CL10 including the CDR sequence(s) of SEQ ID NO: 20 (the DNA sequence thereof is SEQ ID NO: 19).

There were 2 types of anti-CD300c monoclonal antibodies selected using the OPALTL phage library: CB301_OPALTL_B5 including the CDR sequence(s) of SEQ ID NO: 48 (the DNA sequence thereof is SEQ ID NO: 47) and CB301_OPALTL_E6 including the CDR sequence(s) of SEQ ID NO: 50 (the DNA sequence thereof is SEQ ID NO: 49).

From the above results, it was possible to identify 25 types of anti-CD300c monoclonal antibodies that specifically bind, with high binding affinity, to the CD300c antigen and can be used for the prevention or treatment of cancer.

### 1.4. Production and purification of anti-CD300c monoclonal antibody

Using each of the nucleotide sequences of the anti-CD300c monoclonal antibodies identified in Example 1.3, expression vectors capable of expressing each antibody were prepared into which the heavy chain and the light chain are separately inserted. More specifically, the expression vectors were prepared by inserting genes into the pCIW3.3 vectors using the analyzed CDR sequences so that the vectors can express the heavy and light chains, respectively. The prepared expression vectors for heavy and light chains were mixed with polyethylenimine (PEI) in a mass ratio of 1:1 and transfected into 293T cells to induce antibody expression. Then, on day 8, the culture was centrifuged to remove the cells. The resulting culture was obtained. The obtained culture was filtered, and then resuspended using a mixed solution of 0.1 M NaH₂PO₄ and 0.1 M Na₂HPO₄ (pH 7.0). The resuspended solution was purified through affinity chromatography using protein A beads (GE Healthcare), and finally eluted using an elution buffer (Thermofisher).

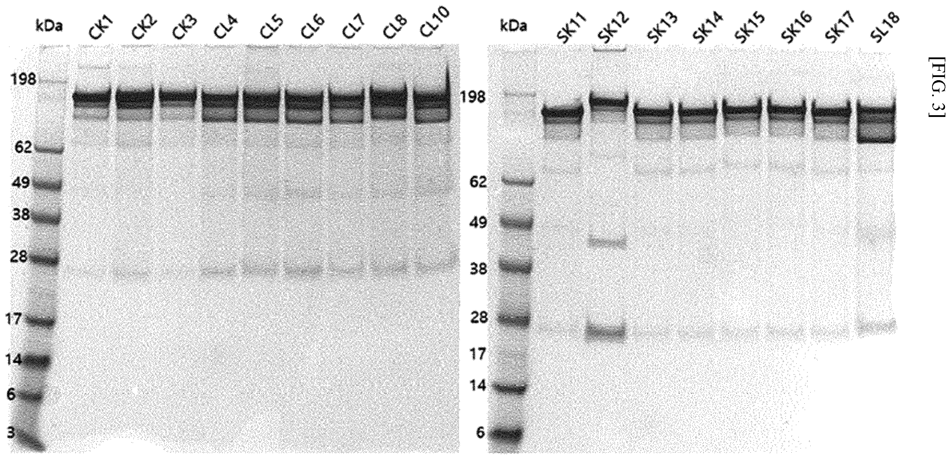

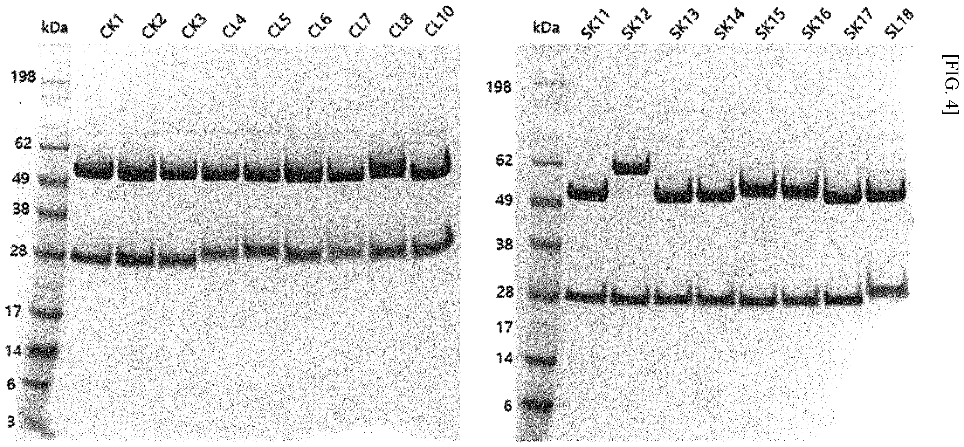

In order to identify the produced antibody, each of reducing sample buffer and non-reducing sample buffer was added to 5 µg of purified antibody, and electrophoresis was performed using pre-made SDS-PAGE (Invitrogen). Then, the proteins were stained using Coomassie Blue. The respective results under a non-reducing condition are illustrated in FIG. 3, and the respective results under a reducing condition are illustrated in FIG. 4.

As illustrated in FIGS. 3 and 4, it was identified that the anti-CD300c monoclonal antibodies having a purity of 90% or higher were produced and purified.

### 1.5. Determination of antigen-binding affinity of anti-CD300c monoclonal antibody

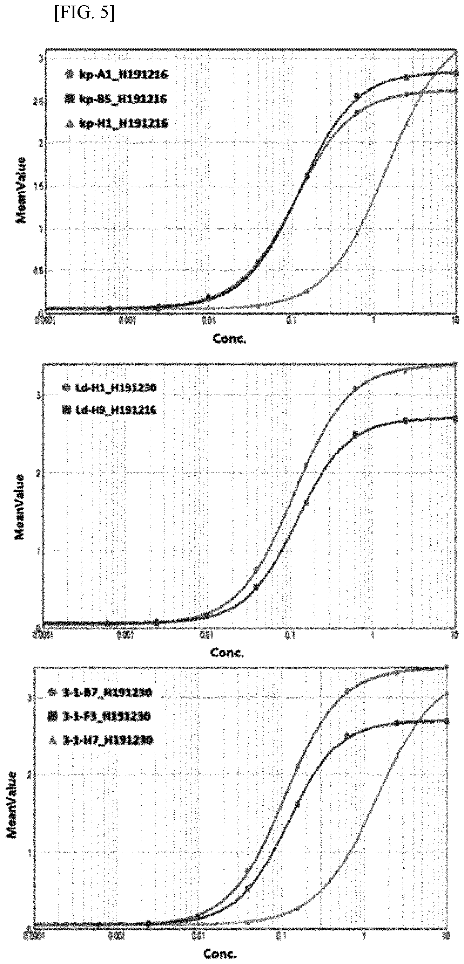

Among the anti-CD300c monoclonal antibodies produced in the same manner as in Example 1.4, binding ELISA was performed to select monoclonal antibodies that specifically bind, with better binding affinity, to the CD300c antigen. More specifically, each of the CD300c antigen or CD300a antigen in a coating buffer (0.1 M sodium carbonate, pH 9.0) was dispensed onto an ELISA plate at a concentration of 8 µg/mL per well, and then reaction was allowed to proceed at room temperature for 3 hours so that the antigen was bound to the plate. Washing was performed 3 times using phosphate buffered saline-Tween 20 (PBST) to remove unbound antigen, and then 300 µL of PBST supplemented with 5% bovine serum albumin (BSA) was added to each well. Reaction was allowed to proceed at room temperature for 1 hour, and washing was performed again using PBST. Then, the anti-CD300c monoclonal antibody was diluted in quadruplicate and added thereto. Reaction was allowed to proceed for 1 hour at room temperature for antigen binding. After 1 hour, washing was performed 3 times using PBST to remove unbound anti-CD300c monoclonal antibody, and then 4 µg/mL of an antibody for detection (HRP conjugated anti-Fc IgG) was added. Reaction was allowed to proceed again at room temperature for 1 hour. Subsequently, the unbound antibody for detection was removed using PBST, and then TMB solution was added. Reaction was allowed to proceed for 10 minutes for development. Then, 2 N sulfuric acid solution was added to terminate the development, and the absorbance was measured at 450 nm to identify the antibodies that specifically bind to the CD300c antigen. The results are shown in Table 1 and FIG. 5.

**[Table 1]**

| CB301 antibody | EC50 (µg/mL) |
|---|---|
| CK1 | 0.056 |
| CK2 | 0.033 |
| CK3 | 0.793 |
| CL4 | 0.031 |
| CL5 | 0.032 |
| CL6 | 0.148 |
| CL7 | 0.047 |
| CL8 | 49.7 |
| CL9 | 0.094 |
| CLIO | 0.039 |
| SK11 | 0.052 |
| SK12 | 0.067 |
| SK13 | 0.044 |
| SK14 | 0.065 |
| SK15 | 14.74 |
| SK16 | 2.42 |
| SK17 | 0.054 |
| SL18 | 0.17 |

As shown in Table 1, as a result of measuring the EC50 (effective concentration of drug that causes 50% of the maximum response) values of the anti-CD300c monoclonal antibodies, it was identified that the remaining all 14 clones except for 4 clones (CK3, CL8, SK15, SK16) exhibited high binding affinity of 0.2 µg/mL or lower.

As illustrated in FIG. 5, it was found that the anti-CD300c monoclonal antibodies of the present disclosure bound to the CD300c antigen with high binding affinity even in the sigmoid curves for the results of the binding ELISA.

### Example 2: Identification of anti-cancer effects of anti-CD300c monoclonal antibody on T cells

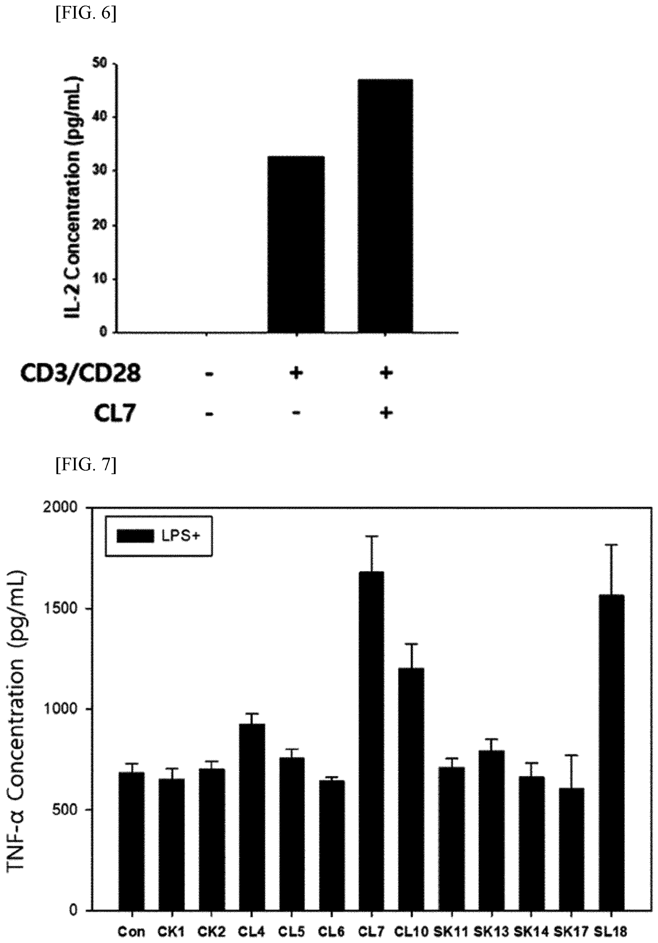

In order to identify whether the anti-CD300c monoclonal antibody selected by the method of Example 1.5 exhibits an anticancer effect by activating T cells, the production level of interleukin-2 (IL-2) was identified. IL-2 is an immune factor that helps growth, proliferation, and differentiation of T cells. Increased production level of IL-2 means activation of T cells due to an increase in stimulation that induces increased differentiation, proliferation, and growth of T cells. More specifically, each of anti-CD3 monoclonal antibody and anti-CD28 monoclonal antibody was added to a 96-well plate at a concentration of 2 µg/well and fixed for 24 hours. Then, co-treatment with 1×10⁵ cells/well of Jurkat T cells (human T lymphocyte cell line) and 10 µg/well of anti-CD300c monoclonal antibody were performed. The production level of IL-2 was measured using an ELISA kit (IL-2 Quantikine kit, R&D Systems), and then compared with the control group that had not been treated with the anti-CD300c monoclonal antibody. The results are illustrated in FIG. 6.

As illustrated in FIG. 6, it was identified that the production level of IL-2 increased in a case where Jurkat T cells activated by treatment with the anti-CD3 monoclonal antibody and the anti-CD28 monoclonal antibody were treated with the anti-CD300c monoclonal antibody. From these results, it was found that the anti-CD300c monoclonal antibody was able to activate T cells, indicating that the anti-CD300c monoclonal antibody can induce anticancer immune action to inhibit growth of cancer tissue.

### Example 3: Identification of anticancer effect by capacity of anti-CD300c monoclonal antibody for causing differentiation into macrophages

### 3.1. Identification of capacity of anti-CD300c monoclonal antibody for causing differentiation into M1 macrophages

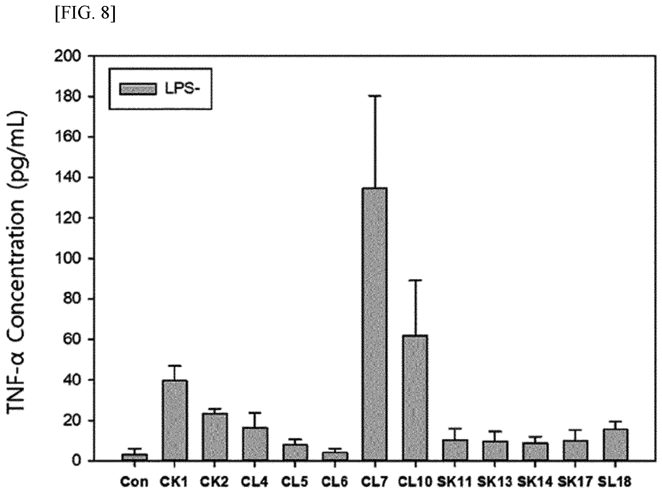

In order to identify that the anti-CD300c monoclonal antibody selected by the method of Example 1.5 induces differentiation of monocytes into M1 macrophages, THP-1 (human monocyte cell line) at 1.5x10⁴ cells/well was dispensed onto a 96-well plate, and treatment with 10 µg/mL of the anti-CD300c monoclonal antibody and/or 100 ng/mL of LPS was performed. Reaction was allowed to proceed for 48 hours, and then the production level of tumor necrosis factor-α (TNF-α), which is a differentiation marker of M1 macrophages, was measured using an ELISA kit (Human TNF-α Quantikine kit, R&D Systems). The results are illustrated in FIGS. 7 and 8.

As illustrated in FIG. 7, it was identified that the anti-CD300c monoclonal antibodies, CL4, CL7, CL10, and SL18, exhibited an increase in production level of TNF-α which is about 2 or more times higher than the control group (Con) treated with LPS alone.

In addition, as illustrated in FIG. 8, it was identified that all the experimental groups treated with the anti-CD300c monoclonal antibody alone without LPS treatment exhibited an increase in production level of TNF-α as compared with the control group (Con) treated with LPS alone.

### 3.2. Identification of differentiation capacity into M1 macrophages depending on concentrations of anti-CD300c monoclonal antibody

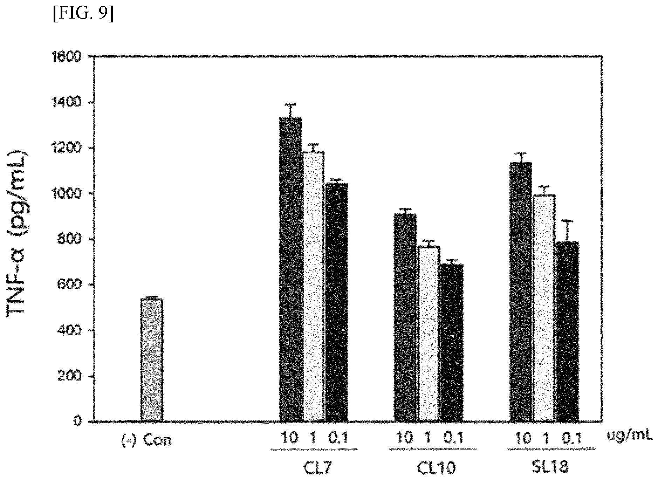

In order to identify that induction of differentiation into M1 macrophages by the anti-CD300c monoclonal antibody increases with concentrations of the anti-CD300c monoclonal antibody, the production level of TNF-α was identified in the same manner as in Example 3.1. Treatment with the anti-CD300c monoclonal antibody was performed at concentrations of 10, 1, and 0.1 µg/mL. The results are illustrated in FIG. 9.

As illustrated in FIG. 9, it was identified that the production level of TNF-α increased as the treatment concentration of the anti-CD300c monoclonal antibody increased.

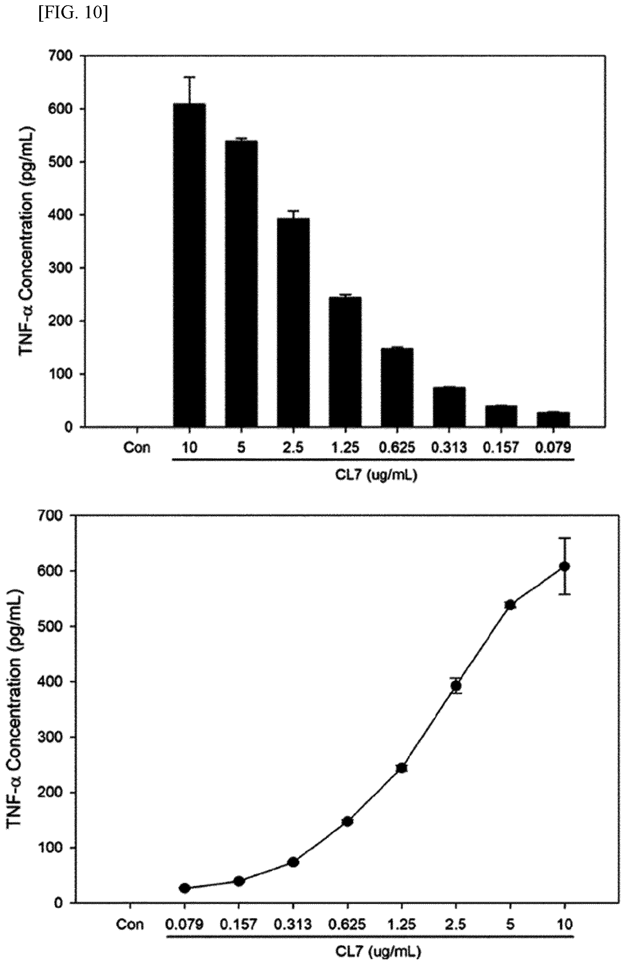

In order to identify results with further divided concentrations, treatment with the anti-CD300c monoclonal antibody CL7 was performed at concentrations of 10, 5, 2.5, 1.25, 0.625, 0.313, 0.157, and 0.079 µg/mL, and the production level ofTNF-α was identified. The results are illustrated in FIG. 10.

As illustrated in FIG. 10, it was identified that the production level of TNF-α increased in a concentration-dependent manner with respect to the anti-CD300c monoclonal antibody.

### 3.3. Identification of differentiation into M1 macrophages caused by anti-CD300c monoclonal antibody through cell shape.

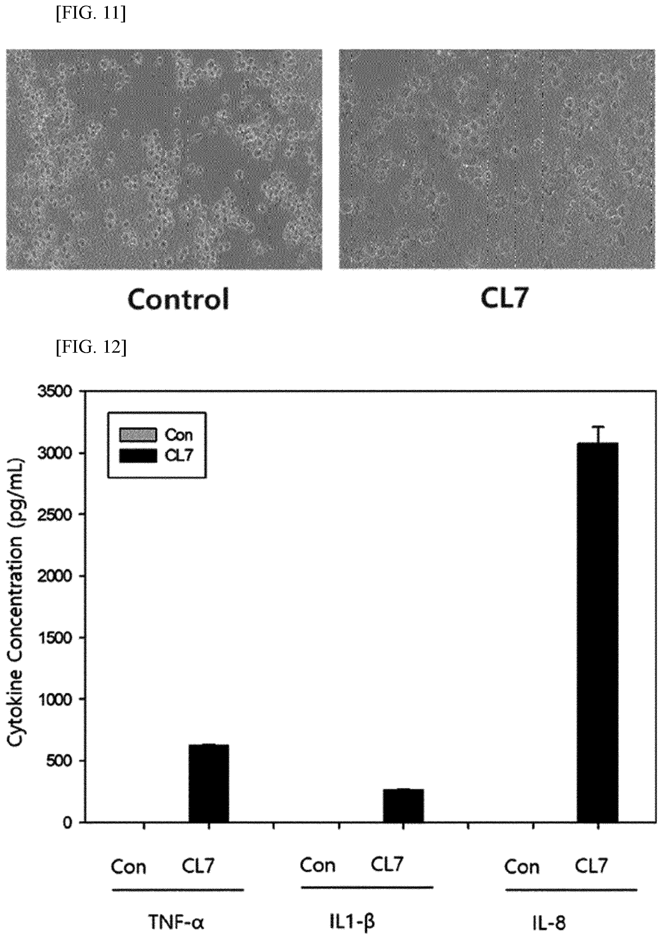

In order to identify, through cell shape, differentiation pattern into M1 macrophages in a case where monocytes are treated with the anti-CD300c monoclonal antibody, THP-1 was treated with 10 µg/mL of the anti-CD300c monoclonal antibody, cultured for 48 hours, and then the shape of the cells was observed under a microscope. The results are illustrated in FIG. 11.

As illustrated in FIG. 11, it was identified that for the experimental group (CL7) treated with the anti-CD300c monoclonal antibody, the shape of THP-1 cells was changed from suspension cells to circular adherent cells that are in the form of M1 macrophages. From these results, it was identified that differentiation of monocytes into M1 macrophages was promoted by treatment with the anti-CD300c monoclonal antibody.

### 3.4. Identification of capacity of anti-CD300c monoclonal antibody CL7 for causing differentiation into M1 macrophages

In order to identify again whether the anti-CD300c monoclonal antibody CL7 promotes differentiation of human monocytes into M1 macrophages, the secretion levels of TNF-α, interleukin-1β (IL-1β), and interleukin-8 (IL-8) were measured using an ELISA kit (R&D Systems). More specifically, THP-1 at 1.5x10⁴ cells/well was dispensed onto a 96-well plate, and treatment with 10 µg/mL of the anti-CD300c monoclonal antibody was performed. Reaction was allowed to proceed for 48 hours, and then the production levels of TNF-α, IL-1β, and IL-8, which are markers for differentiation into M1 macrophages, were measured using an ELISA kit (Human TNF-α Quantikine kit, R&D Systems). The results are illustrated in FIG. 12.

As illustrated in FIG. 12, it was identified that all three types of markers for differentiation into M1 macrophages increased in the experimental group treated with the anti-CD300c monoclonal antibody, as compared with the control group (Con) not treated with the anti-CD300c monoclonal antibody.

### 3.5. Comparison of differentiation capacity into M1 macrophages between anti-CD300c monoclonal antibody and cancer immunotherapy

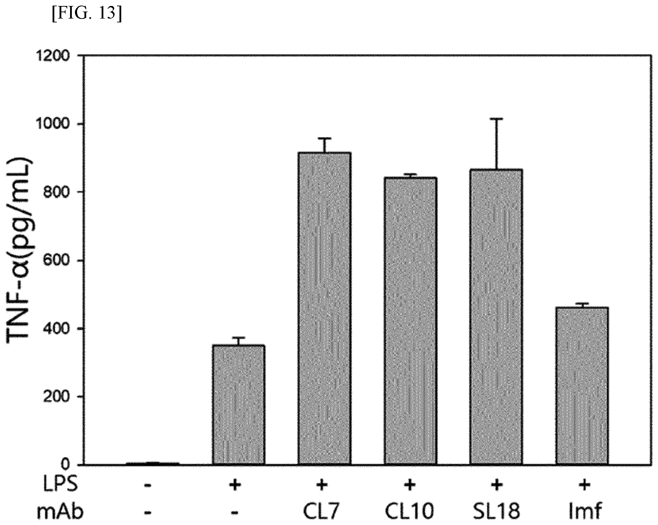

In order to compare differentiation capacity into M1 macrophages between the anti-CD300c monoclonal antibodies and a cancer immunotherapy, the production level of TNF-α was identified using an ELISA kit in the same manner as in Example 3.1. As an anti-PD-Ll immunotherapy, Imfinzi was used at a concentration of 10 µg/mL. The results are illustrated in FIG. 13.

As illustrated in FIG. 13, it was identified that the anti-CD300c monoclonal antibody resulted in a remarkably increased production level of TNF-α as compared with the control group treated with Imfinzi (Imf), which is known as a cancer immunotherapy, alone. From these results, it was found that the anti-CD300c monoclonal antibody resulted in remarkably increased differentiation capacity into M1 macrophages as compared with the conventionally known cancer immunotherapy.

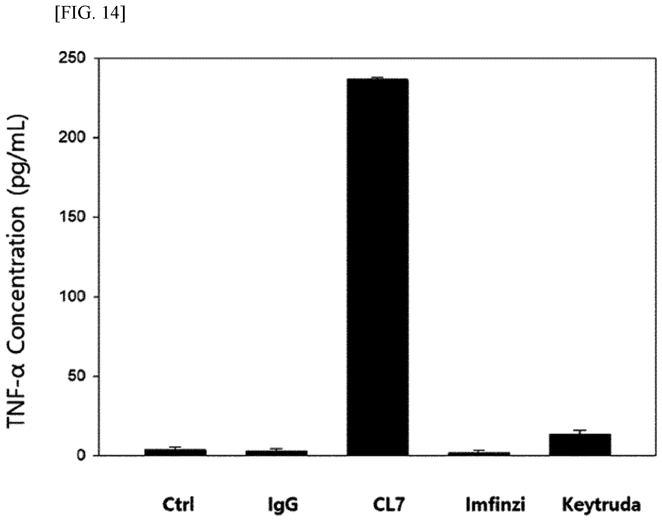

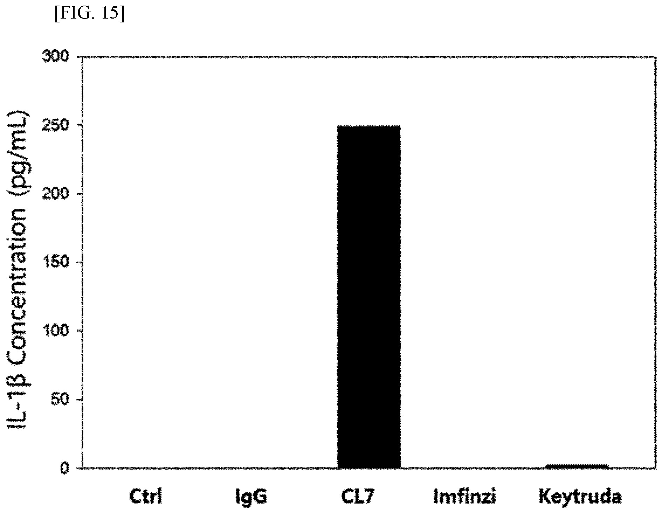

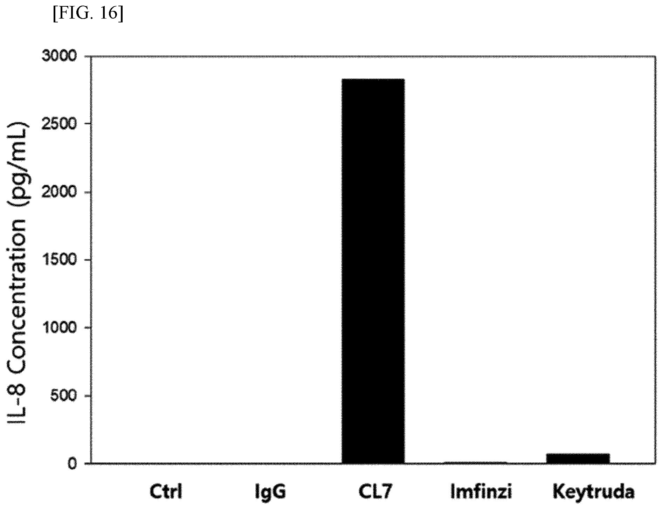

For comparison with other cancer immunotherapies, each of Imfinzi, which is an anti-PD-Ll immunotherapy, Keytruda, which is an anti-PD-1 immunotherapy, and an isotype control (immunoglobulin G) antibody was used at a concentration of 10 (jg/mL, and the production levels of TNF-α, IL-1β, and IL-8 were identified using an ELISA kit. The results are illustrated in FIGS. 14 to 16.

As illustrated in FIGS. 14 to 16, it was identified that the anti-CD300c monoclonal antibody CL7 resulted in remarkably increased production levels of TNF-α, IL-1β, and IL-8 as compared with Imfinzi, Keytruda, and the IgG antibody. From these results, it was found that the anti-CD300c monoclonal antibody was able to result in remarkably increased promotion of differentiation into M1 macrophages as compared with the conventional cancer immunotherapies.

### 3.6. Comparison of differentiation capacity from M0 macrophages into M1 macrophages between anti-CD300c monoclonal antibody and anti-PD-Ll immunotherapy

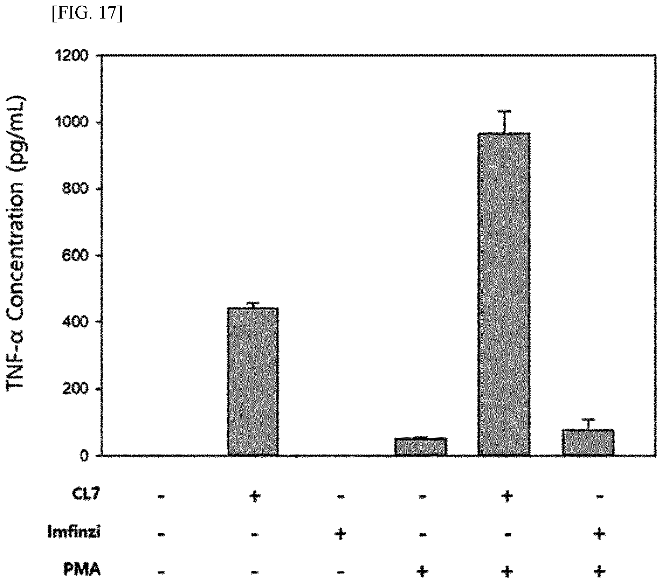

In order to compare differentiation capacity from M0 macrophages into M1 macrophages between the anti-CD300c monoclonal antibodies and cancer immunotherapies, THP-1 at 1.5x10⁴ cells/well was dispensed onto a 96-well plate, and treatment with 10 µg/mL of the anti-CD300c monoclonal antibody, 10 µg/mL of Imfmzi, and/or 200 nM of phorbol-12-myristate-13-acetate (PMA) was performed. Reaction was allowed to proceed for 48 hours, and then the production levels of TNF-α were measured using an ELISA kit. The results are illustrated in FIG. 17.

As illustrated in FIG. 17, it was identified that TNF-α was not produced in the comparative group treated with Imfinzi, which is a cancer immunotherapy, alone, and the production level of TNF-α increased in the experimental group treated with the anti-CD300c monoclonal antibody alone. In addition, it was identified that even in a case where THP-1 was differentiated into M0 macrophages by treatment with PMA, the experimental group treated with the anti-CD300c monoclonal antibody exhibited a remarkably increased production level of TNF-α as compared with the experimental group treated with Imfinzi. From these results, it was found that the anti-CD300c monoclonal antibody promoted differentiation from M0 macrophages into M1 macrophages as compared with a conventional cancer immunotherapy.

### 3.7. Comparison of differentiation capacity into M1 macrophages between anti-CD300c monoclonal antibody and anti-PD-Ll immunotherapy

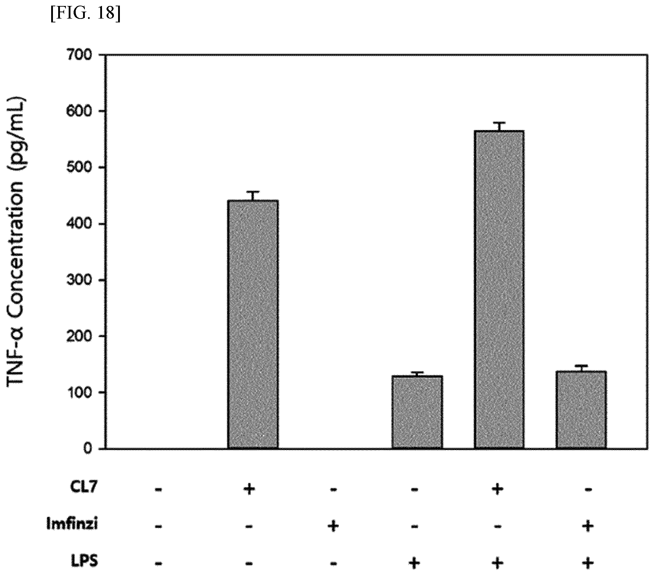

In order to compare differentiation capacity into M1 macrophages between the anti-CD300c monoclonal antibodies and cancer immunotherapies, the production level of TNF-α was identified in the same manner as in Example 3.1. The results are illustrated in FIG. 18.

As illustrated in FIG. 18, it was identified that in a case where monocytes were differentiated into M1 macrophages by treatment with LPS, the experimental group co-treated with Imfinzi and LPS did not exhibit a significant difference in production level of TNF-α, and the experimental group treated with the anti-CD300c monoclonal antibody and LPS exhibited a significant difference in production level of TNF-α as compared with the experimental group treated with the anti-CD300c monoclonal antibody alone.

### 3.8. Identification of redifferentiation capacity from M2 macrophages into M1 macrophages of anti-CD300c monoclonal antibody

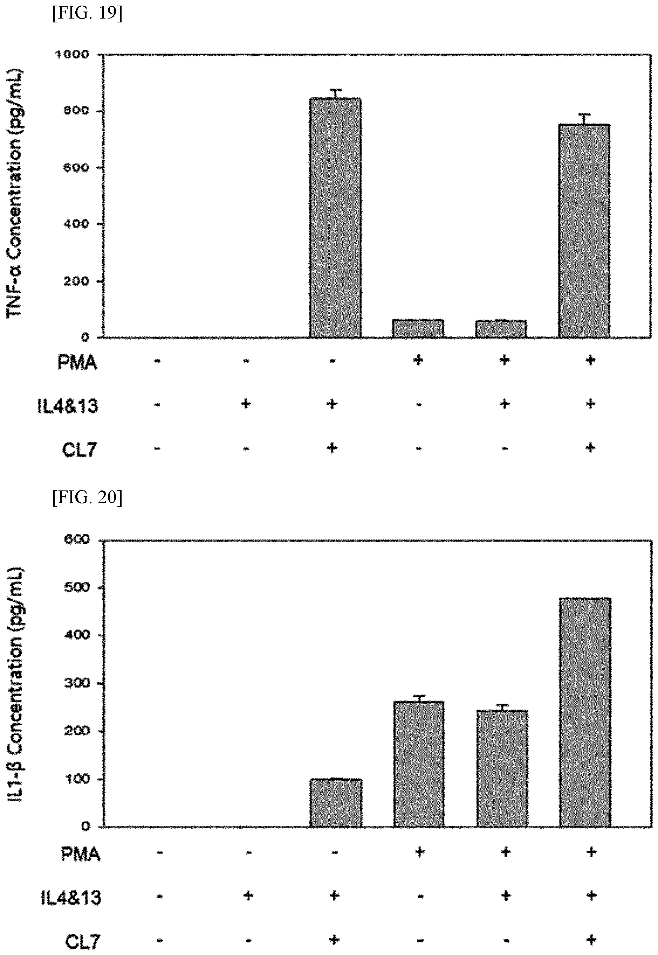

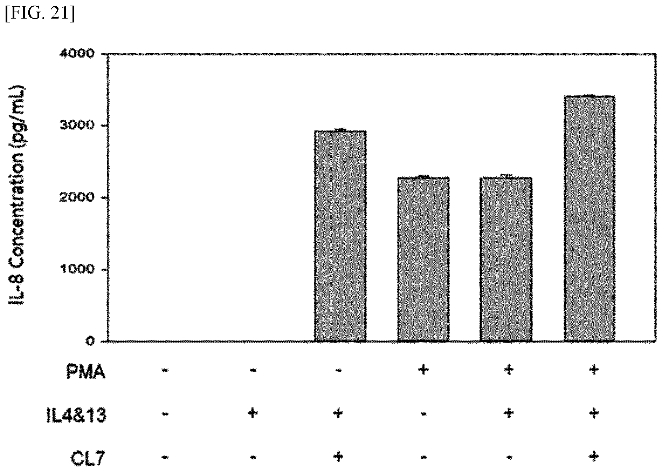

In order to identify that the anti-CD300c monoclonal antibody can redifferentiate M2 macrophages into M1 macrophages, THP-1 at 1.5x10⁴ cells was dispensed onto a 96-well plate, and pre-treated for 6 hours by treatment with 320 nM of PMA. Then, treatment with 20 ng/mL of interleukin-4 (IL-4) and interleukin-13 (IL-13), and with 10 µg/mL of the anti-CD300c monoclonal antibody was performed, and reaction was allowed to proceed for 18 hours. The production levels of TNF-α, IL-1β, and IL-8 were identified using an ELISA kit. The results are illustrated in FIGS. 19 to 21.

As illustrated in FIGS. 19 to 21, it was identified that among the experimental groups not pre-treated with PMA, the experimental group co-treated with IL-4 & IL-13 and the anti-CD300c monoclonal antibody exhibited increased production levels of TNF-α, IL-1β, and IL-8; and among the experimental groups pre-treated with PMA, the experimental group co-treated with IL-4 & IL-13 and the anti-CD300c monoclonal antibody similarly exhibited increased production levels of TNF-α, IL-1β, and IL-8. From these results, it was found that the anti-CD300c monoclonal antibody was able to effectively redifferentiate M2 macrophages into M1 macrophages.

### 3.9. Identification of redifferentiation capacity from M0, M1, and M2 macrophages into M1 macrophages of anti-CD300c monoclonal antibody

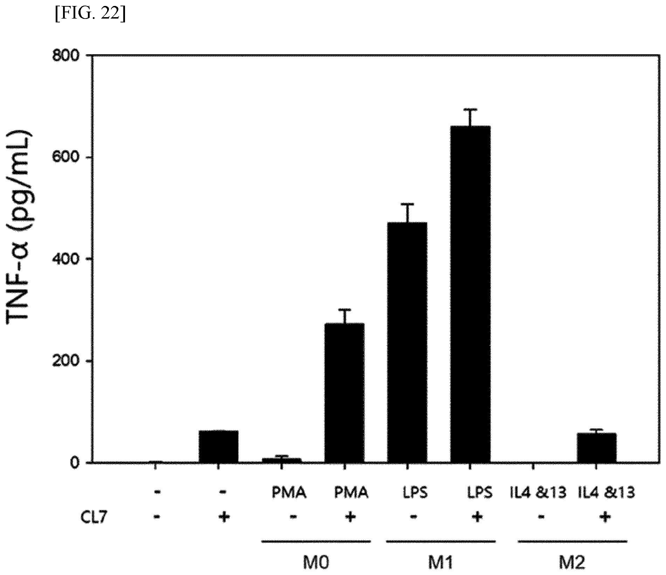

In order to identify that the anti-CD300c monoclonal antibody can redifferentiate M0, M1, and M2 macrophages into M1 macrophages, THP-1 at 1.5x10⁴ cells was dispensed onto a 96-well plate, pre-treated with 10 µg/mL of the anti-CD300c monoclonal antibody for 48 hours, and treated with 100 ng/mL of PMA, 100 ng/mL of LPS, and 20 ng/mL of IL- 4 and IL-13. Reaction was allowed to proceed for 24 hours. The production level of TNF-α was identified using an ELISA kit. The results are illustrated in FIG. 22.

As illustrated in FIG. 22, it was identified that all experimental groups pre-treated with the anti-CD300c monoclonal antibody exhibited a significant increase in production level of TNF-α, as compared with the M0 macrophage control group treated with PMA alone, the M1 macrophage control group treated with LPS alone, and the M2 macrophage control group treated with IL-4 and IL-13 alone. From these results, it was found that the anti-CD300c monoclonal antibody had excellent capacity to differentiate M0, M1, and M2 macrophages into M1 macrophages.

From these results, it was found that the anti-CD300c monoclonal antibody was able to further promote differentiation into M1 macrophages as compared with a conventional cancer immunotherapy, and thus induce anticancer immune action to inhibit growth of cancer tissue. In particular, it was found that the anti-CD300c monoclonal antibody was able to exert an anticancer effect by redifferentiating M2 macrophages, which are known to be involved in promoting proliferation and metastasis of cancer cells, into M1 macrophages.

### Example 4: Identification of effects caused by co-administration of anti-CD300c monoclonal antibody and cancer immunotherapy

### 4.1. Identification of effects caused by co-administration of anti-CD300c monoclonal antibody and anti-PD-Ll immunotherapy

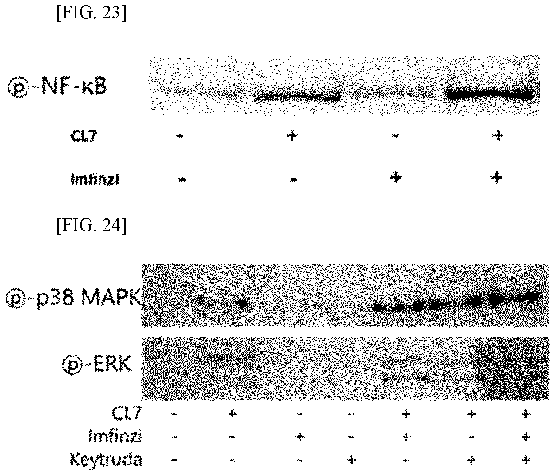

In order to identify the cancer treatment effects caused by co-administration of the anti-CD300c monoclonal antibody and an anti-PD-Ll immunotherapy, NF-κB (nuclear factor kappa-light-chain-enhancer of activated B cells) signal transduction was identified. More specifically, THP-1 at 8.8×10⁵ cells was dispensed onto a 6-well plate, and treated with 10 µg/mL of the anti-CD300c monoclonal antibody CL7 and/or 10 µg/mL of Imfinzi. Incubation was performed for 24 hours, and phosphorylated NF-κB (p-NF-κB) was identified using Western blotting (Cell Signaling Technology). The results are illustrated in FIG. 23.

As illustrated in FIG. 23, it was identified that as compared with the experimental group administered with the immunotherapy Imfinzi alone, the experimental group administered with the anti-CD300c monoclonal antibody exhibited an increased level of p-NF-κB, and the experimental group co-treated with Imfinzi and the anti-CD300c monoclonal antibody exhibited a further increased level in p-NF-κB. From these results, it was found that co-administration of the anti-CD300c monoclonal antibody and Imfinzi promoted differentiation into M1 macrophages.

### 4.2. Identification of effects caused by co-administration of anti-CD300c monoclonal antibody and anti-PD-Ll immunotherapy and/or anti-PD-1 immunotherapy

In order to identify the cancer treatment effects caused by co-administration of the anti-CD300c monoclonal antibody and an anti-PD-Ll immunotherapy and/or an anti-PD-1 immunotherapy, signal transduction of p38 MAPK (p38 mitogen-activated protein kinase) and ERK (extracellular signal-regulated kinase) was identified. More specifically, THP-1 at 8.8x10⁵ cells/well was dispensed onto a 6-well plate, and treated with 10 µg/mL of anti-C300c monoclonal antibody CL7, 10 µg/mL of Imfinzi, and/or 10 µg/mL of Keytruda. Incubation was performed for 48 hours, and phosphorylated p38 MAPK (p-p38 MAPK) and phosphorylated ERK (p-ERK) were identified using Western blotting (Cell Signaling Technology). The results are illustrated in FIG. 24.

As illustrated in FIG. 24, neither p-p38 MAPK nor p-ERK proteins were observed in the experimental group treated with the immunotherapy alone, and both types of proteins were observed in the experimental group treated with the anti-CD300c monoclonal antibody. In addition, it was identified that the level of p-p38 MAPK protein further increased in the experimental groups co-administered with the immunotherapy (ies).

From these results, it was identified that the anti-CD300c monoclonal antibody promoted differentiation into M1 macrophages through MAPK signal transduction and this effect further increased in a case of being co-administered with (an) immunotherapy(ies). Thus, it was found that the anti-CD300c monoclonal antibody was able to be used alone as a cancer immunotherapy, and its anti-cancer therapeutic effects could be further increased through co-administration with a conventional immunotherapy.

### Example 5: Anti-cancer effect in vitro of anti-CD300c monoclonal antibody

### 5.1. Identification of cancer cell growth inhibitory effect of anti-CD300c monoclonal antibody

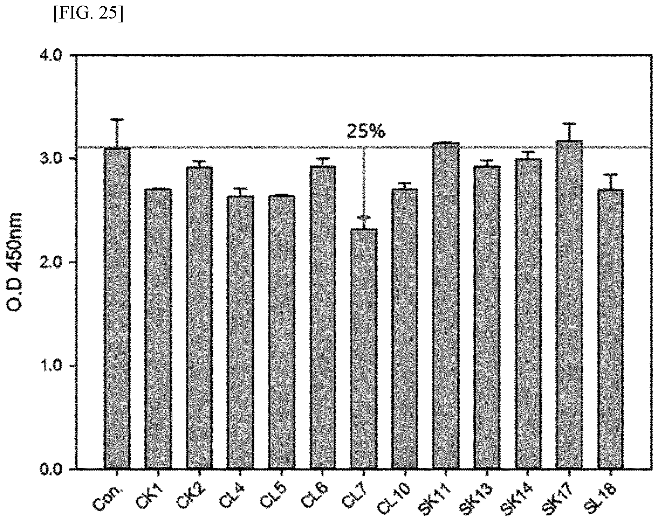

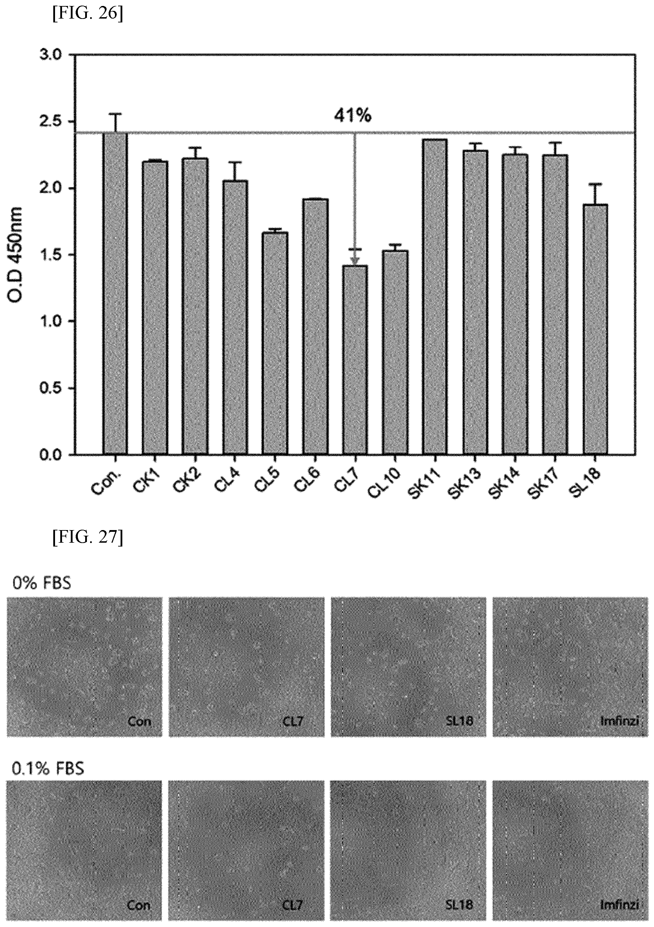

In order to identify effects of the monoclonal antibody, which targets CD300c, on growth of cancer cells, a cell proliferation assay was performed using A549 (human lung cancer cell line). More specifically, onto a 96-well plate were dispensed 2×10⁴ cells under a condition of 0% fetal bovine serum (FBS) and 6×10³ cells under a condition of 0.1% fetal bovine serum. Then, treatment with 10 µg/mL of the anti-CD300c monoclonal antibody was performed and incubation was performed for 5 days. Then, treatment with CCK-8 (DOJINDO) was performed and the absorbance at OD₄₅₀ₙₘ was measured to identify the cancer cell growth inhibitory effects of the anti-CD300c monoclonal antibody. The results are illustrated in FIGS. 25 and 26.

As illustrated in FIG. 25, it was identified that all anti-CD300c monoclonal antibodies except for SK11 and SK14 had an effect of inhibiting proliferation of cancer cells under a condition of 0% FBS.

As illustrated in FIG. 26, it was identified that all anti-CD300c monoclonal antibodies used in the experiment had an effect of inhibiting proliferation of cancer cells under a condition of 0.1% FBS.

### 5.2. Comparison of anti-CD300c monoclonal antibody and cancer cell growth inhibitory effect with cancer immunotherapy

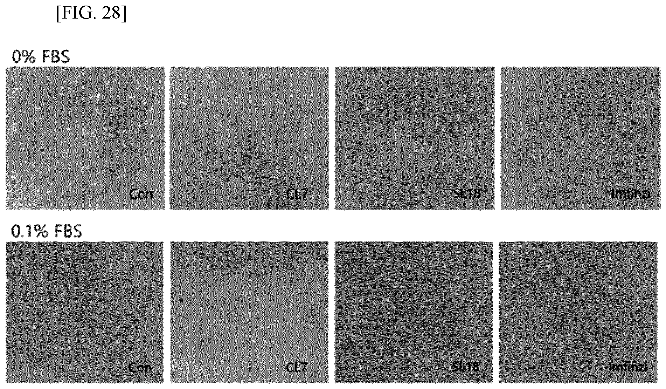

In order to compare the cancer cell growth inhibitory effects between the anti-CD300c monoclonal antibody and a cancer immunotherapy, their cell growth inhibitory effects were identified using A549 (human lung cancer cell line) and MDA-MB-231 (human breast cancer cell line). More specifically, onto a 96-well plate were dispensed 2×10⁴ cells under a condition of 0% fetal bovine serum (FBS) and 6×10³ cells under a condition of 0.1% fetal bovine serum. Treatment with 10 µg/mL of the anti-CD300c monoclonal antibody was performed and incubation was performed for 5 days. Then, observation was made under an optical microscope. The results are illustrated in FIGS. 27 and 28.

As illustrated in FIG. 27, it was identified that the anti-CD300c monoclonal antibody more effectively inhibited proliferation of cancer cells than Imfinzi, which is an immunotherapy, in the A549 cell line.

As illustrated in FIG. 28, it was identified that the anti-CD300c monoclonal antibody more effectively inhibited proliferation of cancer cells than Imfinzi, which is an immunotherapy, in the MDA-MB-231 cell line.

### 5.3. Identification of cancer cell growth inhibitory effects depending on concentrations of anti-CD300c monoclonal antibody

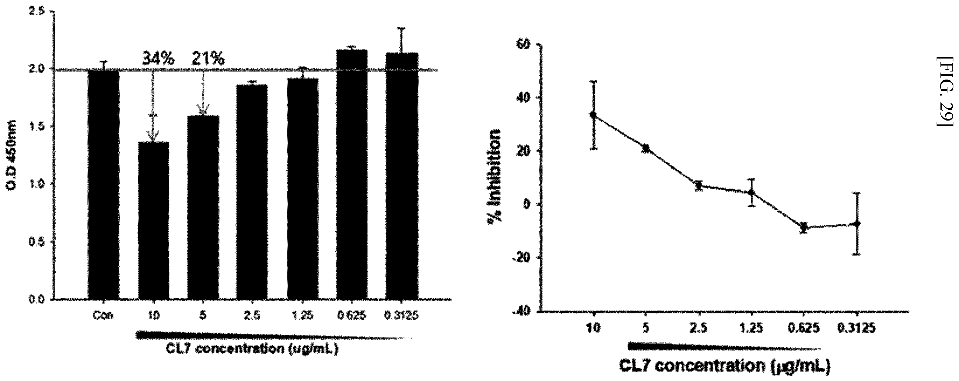

In order to identify the cancer cell growth inhibitory effects depending on concentrations of the anti-CD300c monoclonal antibody, 2×10⁴ A549 cells were dispensed onto a 96-well plate under a condition of 0% fetal bovine serum (FBS), and treatment with 10 µg/mL of the anti-CD300c monoclonal antibody was performed. Incubation was performed for 5 days. Treatment with CCK-8 (DOJINDO) was performed and reaction was allowed to proceed for 3 hours. Then, the absorbance at OD₄₅₀ₙₘ was measured to identify cancer cell growth inhibitory effects of the anti-CD300c monoclonal antibody. The results are illustrated in FIG. 29.

As illustrated in FIG. 29, it was identified that cancer cell growth was inhibited depending on concentrations of the anti-CD300c monoclonal antibody.

### 5.4. Identification of cancer treatment effect by co-administration of anti-CD300c monoclonal antibody and cancer immunotherapy

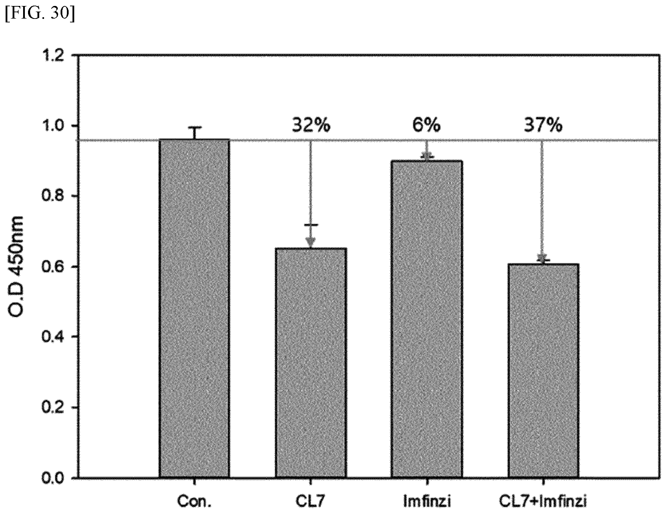

In order to identify the cancer treatment effects caused by co-administration of the anti-CD300c monoclonal antibody and a cancer immunotherapy, a cell proliferation assay was performed in the same manner as in Example 5.1. Imfinzi was used as the immunotherapy. The results are illustrated in FIG. 30.

As illustrated in FIG. 30, it was identified that cancer cell growth was effectively inhibited in a case where the anti-CD300c monoclonal antibody and the immunotherapy were co-administered, as compared with a case where the immunotherapy was administered alone.

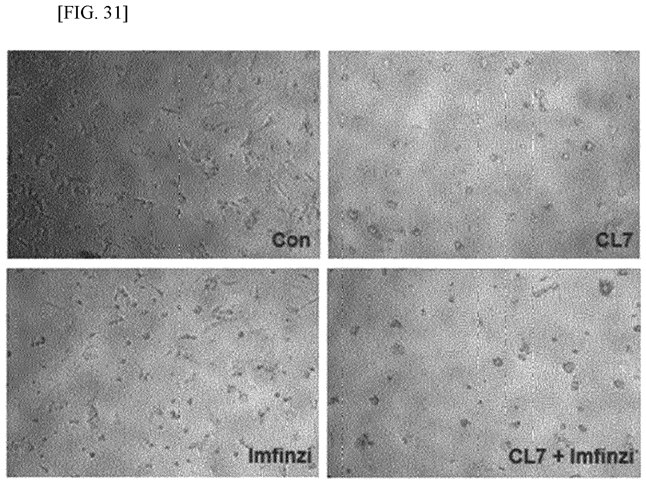

In addition, the cancer cell growth inhibitory effects were observed under an optical microscope. The results are illustrated in FIG. 31.

As illustrated in FIG. 31, it was identified that cancer cell growth was effectively inhibited in a case where the anti-CD300c monoclonal antibody and the immunotherapy were co-administered.

### 5.5. Identification of mechanism of action by co-administration of anti-CD300c monoclonal antibody and cancer immunotherapy

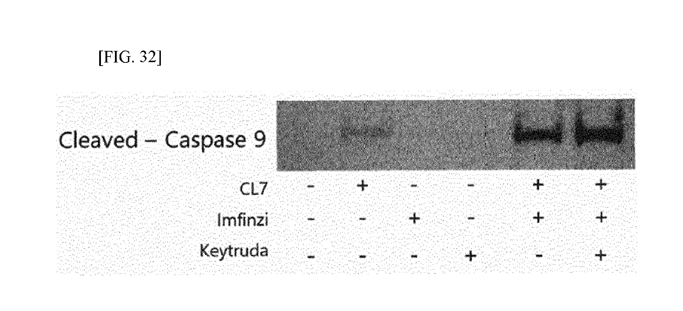

Regarding apoptosis signaling mechanism of cancer cells, in order to identify the mechanism of action by co-administration of the anti-CD300c monoclonal antibody and a cancer immunotherapy, A549 cells were treated with the anti-CD300c monoclonal antibody, Imfinzi, and/or Keytruda, each at a concentration of 10 µg/mL, and levels of cleaved caspase-9, which is an apoptosis marker, was identified by Western blotting (Cell Signaling Technology). The results are illustrated in FIG. 32.

As illustrated in FIG. 32, it was identified that the level of cleaved caspase-9 increased in a case where the anti-CD300c monoclonal antibody and Imfinzi were co-administered, as compared with the experimental group treated with the anti-CD300c monoclonal antibody alone, and the level of cleaved caspase-9 further increased in a case where the anti-CD300c monoclonal antibody, Imfinzi, and Keytruda were co-administered. From these results, it was found that in a case where the anti-CD300c monoclonal antibody and an immunotherapy were co-administered, the apoptosis signaling mechanism of cancer cells was further activated, thereby effectively inhibiting proliferation of cancer cells.

### Example 6: Identification of excellent cross-reactivity of anti-CD300c monoclonal antibody between human and mouse antigens

### 6.1. Identification of specificity of anti-CD300c monoclonal antibody

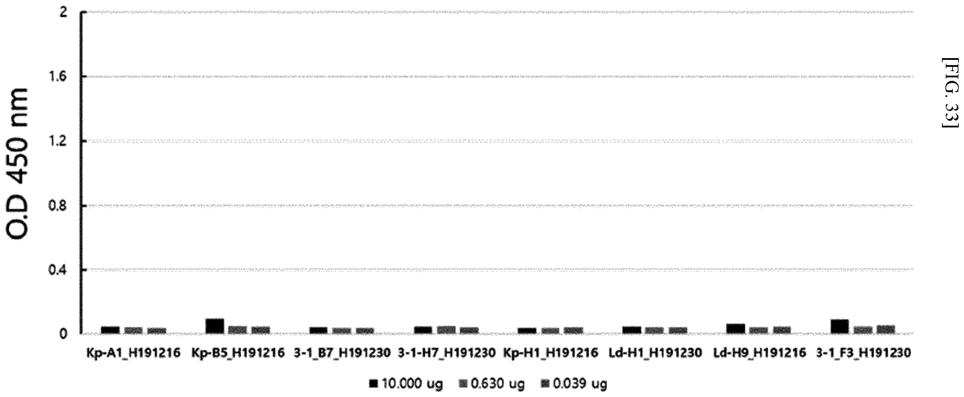

Before identifying cross-reactivity of the anti-CD300c monoclonal antibody between the human and mouse antigens, first, cross-reactivity of the anti-CD300c monoclonal antibody for a CD300a antigen, which is known to antagonize a CD300c antigen and also has a similar protein sequence thereto, was checked to identify specificity of the anti-CD300c monoclonal antibody. More specifically, the anti-CD300c monoclonal antibody was subjected to the CD300a antigen at concentrations of 0.039, 0.63, and 10 µg/mL, and binding ELISA was performed in the same manner as in Example 1.5. The results are illustrated in FIG. 33.

As illustrated in FIG. 33, it was identified that the anti-CD300c monoclonal antibodies did not bind to CD300a. From these results, it was found that the anti-CD300c monoclonal antibodies exhibited high binding specificity to the extent that they do not bind to CD300a that has a similar sequence to CD300c.

### 6.2 Identification of differentiation capacity from mouse macrophages (Raw264.7) into M1 macrophages of anti-CD300c monoclonal antibody

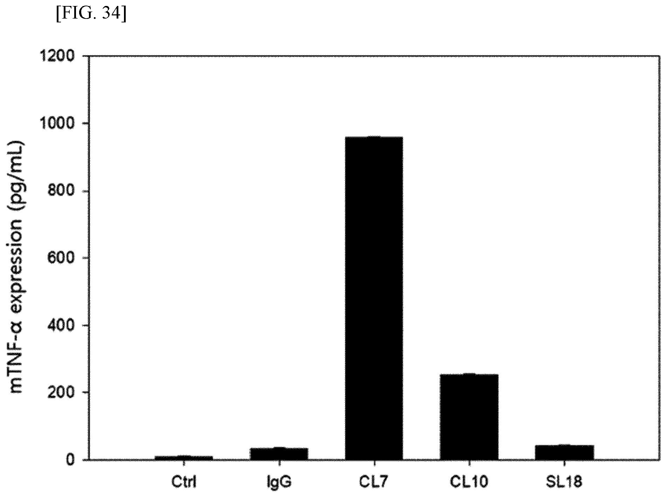

In order to identify that the anti-CD300c monoclonal antibody can even promote differentiation of mouse macrophages into M1 macrophages, 1×10 mouse macrophages (Raw264.7) were dispensed at a concentration of 1×10⁴ cells/well onto a 96-well plate. Then, treatment with 10 µg/mL of the anti-CD300c monoclonal antibody was performed and incubation was performed. The production level of TNF-α was identified using an ELISA kit. The results are illustrated in FIG. 34.

As illustrated in FIG. 34, it was identified that the production level of TNF-α increased in the experimental groups treated with the anti-CD300c monoclonal antibody. From these results, it was found that the anti-CD300c monoclonal antibody promoted differentiation into M1 macrophages by exerting the same action in mice as well as in humans.

### 6.3 Growth inhibitory effects of anti-CD300c monoclonal antibody on mouse colorectal cancer cells (CT26)

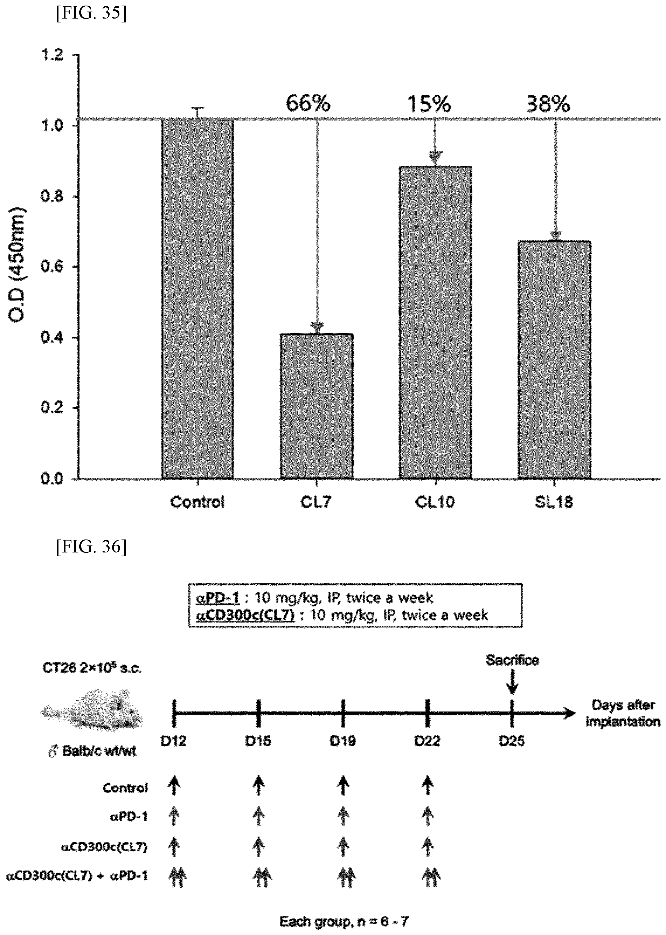

In order to identify whether the anti-CD300c monoclonal antibody exhibits an anticancer effect even in mice, a cell proliferation assay was performed in the same manner as in Example 4.1 using CT26 (mouse colorectal cancer cell line). The results are illustrated in FIG. 35.

As illustrated in FIG. 35, it was identified that the anti-CD300c monoclonal antibodies exhibited a cancer treatment effect even in mice.

### Example 7: Anti-cancer effects in vivo of anti-CD300c monoclonal antibody

### 7.1. Identification of cancer growth inhibitory effects in vivo of anti-CD300c monoclonal antibody

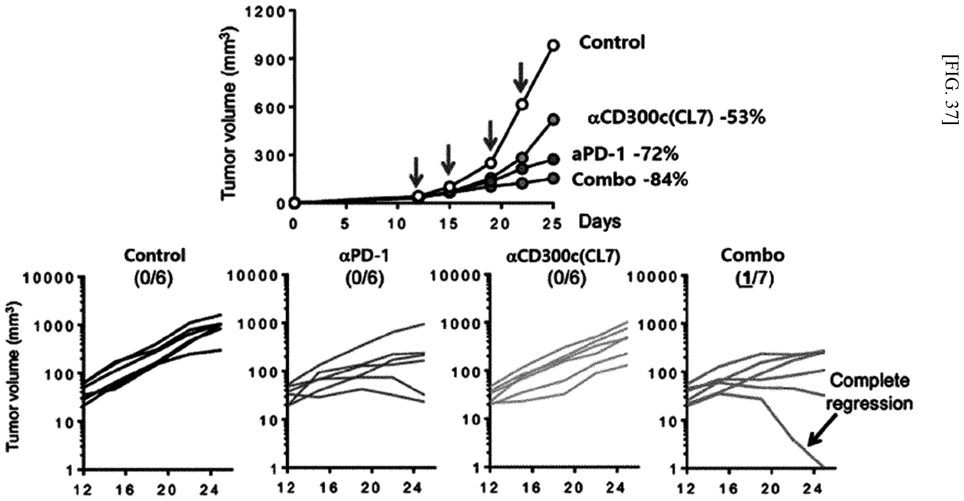

In order to identify anticancer effects *in vivo* of the anti-CD300c monoclonal antibody, a colorectal cancer cell line (CT26) at 2×10⁵ cells was transplanted by subcutaneous injection into 8-week-old BALB/c mice to prepare a mouse syngeneic tumor model. Breeding and experiments for animals were all conducted in a specific pathogen free (SPF) facility. The experimental method is briefly illustrated in FIG. 36. 12 days after transplantation of the colorectal cancer cell line, the mice with tumor size of 50 to 100 mm³ were injected with the anti-CD300c monoclonal antibody CL7, an anti-PD-1 antibody, and both CL7 and the anti-PD-1 antibody (Combo), respectively, and injected with an equal amount of phosphate buffered saline (PBS) as a control group. More specifically, the mice were intraperitoneally injected with 10 mg/kg of each material twice a week for two weeks (a total of 4 times). Then, the tumor volume was measured for 25 days. The results are illustrated in FIG. 37.

As illustrated in FIG. 37, it was identified that cancer growth was inhibited in the experimental group administered with the anti-CD300c monoclonal antibody alone, as compared with the control group, and that cancer growth was further effectively inhibited in a case where the anti-CD300c monoclonal antibody and the anti-PD-1 antibody were co-administered.

### 7.2. Identification of effects of anti-CD300c monoclonal antibody on increase in tumor-infiltrating lymphocytes under tumor microenvironment in vivo

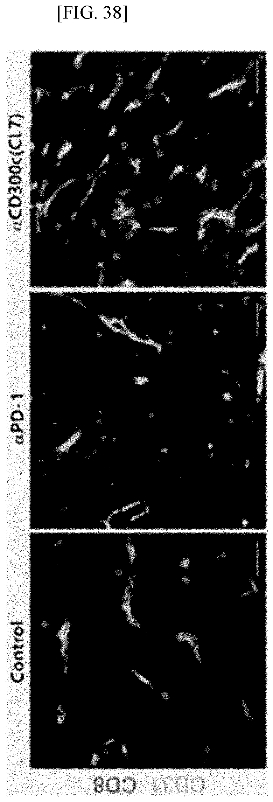

In order to identify the effects of the anti-CD300c monoclonal antibody on tumor-infiltrating lymphocytes (TIL) in a tumor microenvironment (TME), on day 25 of the experiment performed in the same manner as in Example 7.1, the mice were euthanized, 1% paraformaldehyde (PFA) was intravascularly injected thereinto, and then perfusion was performed to obtain cancer tissue. The obtained cancer tissue was fixed using 1% PFA, and sequentially dehydrated using 10%, 20%, and 30% sucrose solution. The dehydrated cancer tissue was frozen in OCT compound (optimal cutting temperature compound), and then the cancer tissue was sectioned to a thickness of 50 µm using a cryotome. Staining of CD8⁺ T cells and CD31⁺ cancer vascular cells, which are tumor-infiltrating lymphocyte markers, was performed. The results are illustrated in FIG. 38.

As illustrated in FIG. 38, it was identified that the experimental group administered with the anti-CD300c monoclonal antibody exhibited an increased level of CD8⁺ cells as compared with the experimental group administered with the anti-PD-1 antibody alone. From these results, it was found that the CD300c monoclonal antibody increased tumor-infiltrating lymphocytes in a tumor microenvironment, thereby exhibiting an anticancer effect.

### 7.3. Identification of M1 macrophage increase effect in vivo of anti-CD300c monoclonal antibody

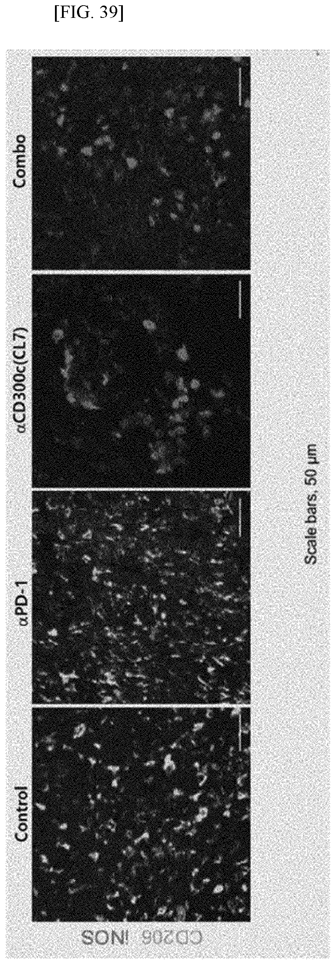

In order to identify whether the anti-CD300c monoclonal antibody increases M1 macrophages in cancer tissue *in vivo,* staining of iNOS, which is an M1 macrophage marker, and CD206, which is an M2 macrophage marker, was performed with the cancer tissue section prepared in the same manner as in Example 7.2. The results are illustrated in FIG. 39.

As illustrated in FIG. 39, it was identified that as compared with the control group, the experimental group treated with the anti-PD-1 antibody exhibited a partially increased level of M1 macrophages and the experimental group treated with the anti-CD300c monoclonal antibody exhibited a remarkably increased level of M1 macrophages with almost no observable M2 macrophages. In addition, it was identified that the experimental group co-administered with the anti-CD300c monoclonal antibody and the anti-PD-1 antibody exhibited an increased level of M1 macrophages. From these results, it was found that the anti-CD300c monoclonal antibody was able to effectively promote differentiation into M1 macrophages as compared with a conventional cancer immunotherapy.

Consequently, it was identified that the anti-CD300c monoclonal antibody of the present disclosure was able to bind, with high specificity, to a CD300c antigen and exhibit inter-species cross-reactivity for, for example, mice, indicating that the anti-CD300c monoclonal antibody can be used for various individuals. In addition, it was identified both *in vitro* and *in vivo* that the anti-CD300c monoclonal antibody was able to serve as a cancer immunotherapy by activating T cells and promoting differentiation into M1 macrophages, thereby effectively inhibiting proliferation, metastasis, or the like of cancer cells, and it was identified that the anti-CD300c monoclonal antibody was able to exhibit a further increased therapeutic effect through co-administration with the conventional cancer immunotherapy. Thus, it was found that the anti-CD300c monoclonal antibody was able to be effectively used for anticancer immunotherapy of various cancers expressing a CD300c antigen.

The description of the present disclosure as described above is provided for illustration, and those of ordinary skill in the art to which the present disclosure pertains will be able to understand that the embodiments disclosed herein can be easily modified into other specific forms without changing the technical spirit or essential features of the present disclosure. Therefore, it should be understood that the embodiments are illustrative and not restrictive in all respects.

### Industrial Applicability

The anti-CD300c monoclonal antibody of the present disclosure specifically binds to CD300c expressed on the surface of cancer cells, and thus can be used for the treatment of any cancer that secretes the CD300c antigen. Also, the anti-CD300c monoclonal antibody has inter-species cross-reactivity, and thus can be used for anticancer treatment of various individuals. In addition, since the anti-CD300c monoclonal antibody exhibits an anticancer effect by serving as a cancer immunotherapy, it can effectively inhibit proliferation, development, metastasis, or the like of cancer. This allows the anti-CD300c monoclonal antibody to be effectively used for immunotherapy of various cancers.

## Claims

1. An anti-CD300c monoclonal antibody, comprising:
any one or more complementarity-determining region (CDR) sequences selected from the group consisting of SEQ ID NOs: 2, 4, 6, 8, 10, 12, 14, 16, 18, 20, 22, 24, 26, 28, 30, 32, 34, 36, 38, 40, 42, 44, 46, 48, and 50.

2. The anti-CD300c monoclonal antibody of claim 1, wherein the anti-CD300c monoclonal antibody has inter-species cross-reactivity.

3. The anti-CD300c monoclonal antibody of claim 2, wherein the cross-reactivity is cross-reactivity between a human antigen and a mouse antigen.

4. A pharmaceutical composition for preventing or treating cancer, comprising an anti-CD300c monoclonal antibody as an active ingredient.

5. The pharmaceutical composition of claim 4, wherein the antibody comprises any one or more complementarity-determining region (CDR) sequences selected from the group consisting of SEQ ID NOs: 2, 4, 6, 8, 10, 12, 14, 16, 18, 20, 22, 24, 26, 28, 30, 32, 34, 36, 38, 40, 42, 44, 46, 48, and 50.

6. The pharmaceutical composition of claim 4, wherein the cancer is any one or more selected from the group consisting of colorectal cancer, rectal cancer, colon cancer, thyroid cancer, oral cancer, pharyngeal cancer, laryngeal cancer, cervical cancer, brain cancer, lung cancer, ovarian cancer, bladder cancer, kidney cancer, liver cancer, pancreatic cancer, prostate cancer, skin cancer, tongue cancer, breast cancer, uterine cancer, stomach cancer, bone cancer, and blood cancer.

7. The pharmaceutical composition of claim 4, further comprising a cancer immunotherapy.

8. The pharmaceutical composition of claim 7, wherein the cancer immunotherapy is any one or more selected from the group consisting of anti-PD-1, anti-PD-Ll, anti-CTLA-4, anti-KIR, anti-LAG3, anti-CD 137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, and anti-TIGIT.

9. The pharmaceutical composition according to claim 4, wherein the pharmaceutical composition inhibits proliferation, survival, metastasis, recurrence, or therapy resistance of cancer.

10. A cancer immunotherapy, comprising an anti-CD300c monoclonal antibody as an active ingredient.

11. The cancer immunotherapy of claim 10, wherein the antibody includes any one or more complementarity-determining region (CDR) sequences selected from the group consisting of SEQ ID NOs: 2, 4, 6, 8, 10, 12, 14, 16, 18, 20, 22, 24, 26, 28, 30, 32, 34, 36, 38, 40, 42, 44, 46, 48, and 50.

12. The cancer immunotherapy of claim 10, wherein the cancer immunotherapy inhibits proliferation, survival, metastasis, recurrence, or therapy resistance of cancer.

13. The cancer immunotherapy of claim 10, wherein the cancer is any one or more selected from the group consisting of colorectal cancer, rectal cancer, colon cancer, thyroid cancer, oral cancer, pharyngeal cancer, laryngeal cancer, cervical cancer, brain cancer, lung cancer, ovarian cancer, bladder cancer, kidney cancer, liver cancer, pancreatic cancer, prostate cancer, skin cancer, tongue cancer, breast cancer, uterine cancer, stomach cancer, bone cancer, and blood cancer.

14. An adjuvant for anticancer therapy, comprising an anti-CD300c monoclonal antibody as an active ingredient.

15. The adjuvant of claim 14, wherein the adjuvant activates an immune function of immune cells to result in an enhanced anticancer therapeutic effect.

16. The adjuvant of claim 14, wherein the anticancer therapy is radiation therapy, chemotherapy, or immunotherapy.

17. A method for preventing or treating cancer, comprising:
a step of administering to an individual a composition that comprises an anti-CD300c monoclonal antibody as an active ingredient.

18. A use of a composition, which comprises an anti-CD300c monoclonal antibody as an active ingredient, for preventing or treating cancer.


<110>    CentricsBio

<120>    Composition for preventing or treating of cancer comprising
         anti-CD300c monoclonal antibody

<130>    MP19-292P1

<150>    KR 10-2019-0147496
<151>    2019-11-18

<150>    KR 10-2020-0154076
<151>    2020-11-17

<160>    50

<170>    KoPatentIn 3.0

<210>    1
<211>    714
<212>    DNA
<213>    Artificial Sequence

<220>
<223>    CK1


<400>    1
gaagtgcagc tgctggaaag tggaggtgga ctggtgcagc ctggcggcag cctgcgcctg        60

agctgtgccg ccagcggatt caccttcagc cgctatgcca tgacctgggt tcgccaagca       120

cctggcaaag gcctggaatg ggtgagcagc atgagcggca ccggcggcac cacctattat       180

gccgatagcg tgaaaggtcg ctttaccatc agccgcgata acagcaaaaa caccctgtat       240

ctgcagatga acagcctgcg cgccgaggac accgcagtct actactgtgc ccgcggcgcc       300

tatggctttg atcattgggg acaaggtact ctggtgaccg tgagcagcag tggaggaggt       360

agcggaggtg gtggatctgg aggtggaggt agtgaaatcg tgctgaccca gagccctggc       420

accctgagcc tgagccctgg cgaacgcgca acactgtcat gccgcgccag ccagagcatc       480

ggcaactatc tgaactggta tcagcagaaa ccaggtcagg ctccacgtct gctgatctat       540

gatgccagca acctggaaac cggcatccct gatcgcttct caggatctgg aagcggtacc       600

gattttaccc tgaccatcag ccgcctggaa cctgaggact ttgccgtgta ttattgtcag       660

cagagtagcg ccatccctta taccttcggt cagggcacta aagtggaaat caaa             714


<210>    2
<211>    238
<212>    PRT
<213>    Artificial Sequence

<220>
<223>    CK1


<400>    2
Glu Val Gln Leu Leu Glu Ser Gly Gly Gly Leu Val Gln Pro Gly Gly

1                   5                   10                  15

Ser Leu Arg Leu Ser Cys Ala Ala Ser Gly Phe Thr Phe Ser Arg Tyr
            20                  25                  30

Ala Met Thr Trp Val Arg Gln Ala Pro Gly Lys Gly Leu Glu Trp Val
        35                  40                  45

Ser Ser Met Ser Gly Thr Gly Gly Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Ala Tyr Gly Phe Asp His Trp Gly Gln Gly Thr Leu Val
            100                 105                 110

Thr Val Ser Ser Ser Gly Gly Gly Ser Gly Gly Gly Gly Ser Gly Gly
        115                 120                 125

Gly Gly Ser Glu Ile Val Leu Thr Gln Ser Pro Gly Thr Leu Ser Leu
    130                 135                 140

Ser Pro Gly Glu Arg Ala Thr Leu Ser Cys Arg Ala Ser Gln Ser Ile
145                 150                 155                 160

Gly Asn Tyr Leu Asn Trp Tyr Gln Gln Lys Pro Gly Gln Ala Pro Arg
                165                 170                 175

Leu Leu Ile Tyr Asp Ala Ser Asn Leu Glu Thr Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Ser Ser Ala
    210                 215                 220

Ile Pro Tyr Thr Phe Gly Gln Gly Thr Lys Val Glu Ile Lys
225                 230                 235


<210>   3
<211>   720
<212>   DNA
<213>   Artificial Sequence

<220>
<223>   CK2


<400>   3
gcggccgaag tgcagctgct ggaaagtgga ggtggactgg tgcagcctgg cggcagcctg        60

cgcctgagct gtgccgccag cggattcacc ttcagcagct atggcatgca ttgggttcgc       120

caagcacctg gcaaaggcct ggaatgggtg agcgccatca gcggcagcgg caccagcatc       180

tattatgccg atagcgtgaa aggccgcttt accatcagcc gcgataacag caaaaacacc       240

ctgtatctgc agatgaacag cctgcgcgcc gaggacaccg cagtctacta ctgtgcccgc 300

ggcggcaccg cctttgatta ttggggacaa ggtactctgg tgaccgtgag cagcggtgga 360

ggaggtagcg gaggtggtgg atctggaggt ggaggtagtg aaatcgtgct gacccagagc 420

cctggcaccc tgagcctgag ccctggcgaa cgcgcaacac tgtcatgccg cgccagccag 480

agatcagaca actatctggc ctggtatcag cagaaaccag gtcaggctcc acgtctgctg 540

atctatgatg ccagcaaccg cgccaccggc atccctgatc gcttctcagg atctggaagc 600

ggtaccgatt ttaccctgac catcagccgc ctggaacctg aggactttgc cgtgtattat 660

tgtcagcaga gctatagcac cccttttacc ttcggtcagg gcactaaagt ggaaaccaaa 720

720

<210> 4
<211> 238
<212> PRT
<213> Artificial Sequence

<220>
<223> CK2

<400> 4
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ser Ala Ile Ser Gly Ser Gly Thr Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Gly Thr Ala Phe Asp Tyr Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Glu Ile Val Leu Thr Gln Ser Pro Gly Thr Leu Ser Leu
    130                 135                 140

Ser Pro Gly Glu Arg Ala Thr Leu Ser Cys Arg Ala Ser Gln Arg Ser
145                 150                 155                 160

Asp Asn Tyr Leu Ala Trp Tyr Gln Gln Lys Pro Gly Gln Ala Pro Arg
                165                 170                 175

Leu Leu Ile Tyr Asp Ala Ser Asn Arg Ala Thr Gly Ile Pro Asp Arg

180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Ser Tyr Ser
    210                 215                 220

Thr Pro Phe Thr Phe Gly Gln Gly Thr Lys Val Glu Thr Lys
225                 230                 235

<210>    5
<211>    723
<212>    DNA
<213>    Artificial Sequence

<220>
<223>    CK3

<400>    5
ggccgagtgc agctgctgga aagtggaggt ggactggtgc agcctggcgg cagcctgcgc      60

ctgagctgtg ccgccagcgg attcaccttc agcagctatg ccatcagctg ggttcgccaa     120

gcacctggca aaggcctgga atgggtgagc gccaccagcg gcagcggccg cgccacctat     180

tatgccgata gcgtgaaagg ccgctttacc atcagccgcg ataacagcaa aaacaccctg     240

tatctgcaga tgaacagcct gcgcgccgag gacaccgcag tctactactg tgcgcgcgat     300

acctggtggg aaggctattt tgatctgtgg ggacaaggta ctctggtgac cgtgagcagc     360

agtggaggag gtagcggagg tggtggatct ggaggtggag gtagtgaaat cgtgctgacc     420

cagagccctg gcaccctgag cctgagccct ggcgaacgcg caacactgtc atgccaggcc     480

agccatatca gcacccatct gaactggtat cagcagaaac caggtcaggc tccacgtctg     540

ctgatctatg gcgccagcag ccgcgccacc ggcatccctg atcgcttctc aggatctgga     600

agcggtaccg attttaccct gaccatcagc cgcctggaac ctgaggactt tgccgtgtat     660

tattgtcagc agtataacac ctatcctcct accttcggtc agggcactaa agtggaaatc     720

aaa                                                                   723

<210>    6
<211>    240
<212>    PRT
<213>    Artificial Sequence

<220>
<223>    CK3

<400>    6
Arg Val Gln Leu Leu Glu Ser Gly Gly Gly Leu Val Gln Pro Gly Gly
 1               5                  10                  15

Ser Leu Arg Leu Ser Cys Ala Ala Ser Gly Phe Thr Phe Ser Ser Tyr

20                  25                  30

Ala Ile Ser Trp Val Arg Gln Ala Pro Gly Lys Gly Leu Glu Trp Val
        35                  40                  45

Ser Ala Thr Ser Gly Ser Gly Arg Ala Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Thr Trp Trp Glu Gly Tyr Phe Asp Leu Trp Gly Gln Gly
            100                 105                 110

Thr Leu Val Thr Val Ser Ser Ser Gly Gly Gly Ser Gly Gly Gly Gly
        115                 120                 125

Ser Gly Gly Gly Gly Ser Glu Ile Val Leu Thr Gln Ser Pro Gly Thr
    130                 135                 140

Leu Ser Leu Ser Pro Gly Glu Arg Ala Thr Leu Ser Cys Gln Ala Ser
145                 150                 155                 160

His Ile Ser Thr His Leu Asn Trp Tyr Gln Gln Lys Pro Gly Gln Ala
                165                 170                 175

Pro Arg Leu Leu Ile Tyr Gly Ala Ser Ser Arg Ala Thr Gly Ile Pro
            180                 185                 190

Asp Arg Phe Ser Gly Ser Gly Ser Gly Thr Asp Phe Thr Leu Thr Ile
        195                 200                 205

Ser Arg Leu Glu Pro Glu Asp Phe Ala Val Tyr Tyr Cys Gln Gln Tyr
    210                 215                 220

Asn Thr Tyr Pro Pro Thr Phe Gly Gln Gly Thr Lys Val Glu Ile Lys
225                 230                 235                 240


<210>   7
<211>   705
<212>   DNA
<213>   Artificial Sequence

<220>
<223>   CL4


<400>   7
cgagtgcagc tgctggaaag tggaggtgga ctggtgcagc ctggcggcag cctgcgcctg      60

agctgtgccg ccagcggatt caccttcggc agcaactata tgagctgggt tcgccaagca     120

cctggcaaag gcctggaatg ggtgagcacc atcagcggca gcggcaccag cacctattat     180

gccgatagct tgaaaggccg ctttaccatc agccgcgata acagcaaaaa caccctgtat     240

ctgcagatga acagcctgcg cgccgaggac accgcagtct actactgtgc ccgcggcatg     300

tggggcatgg atgtgtgggg acaaggtact ctggtgaccg tgagcagcgg tggaggaggt     360

agcggaggtg gtggatctgg aggtggaggt agtcagagcg tgctgaccca gcctcctagc 420

gcctccggta caccaggaca gcgcgtgact attagctgta ccggcaaaca tcggcacacc 480

gtgaactggt accagctact gcctggaact gcacctaagc tgctgatcta tctggatagc 540

gaacgcccta gcggcgtacc tgatcgcttt agcggtagca aatcaggcac cagcgccagc 600

ctggccatca gcggccttcg ctccgaagat gaagccgatt attattgtca gagctatgat 660

agcagcagcg tggtgtttgg tggcggtacc aagctgaccg tgctg 705

<210> 8
<211> 235
<212> PRT
<213> Artificial Sequence

<220>
<223> CL4

<400> 8
Arg Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Gly Ser Asn
            20                  25                  30

Tyr Met Ser Trp Val Arg Gln Ala Pro Gly Lys Gly Leu Glu Trp Val
        35                  40                  45

Ser Thr Ile Ser Gly Ser Gly Thr Ser Thr Tyr Tyr Ala Asp Ser Leu
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Met Trp Gly Met Asp Val Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Gln Ser Val Leu Thr Gln Pro Pro Ser Ala Ser Gly Thr
    130                 135                 140

Pro Gly Gln Arg Val Thr Ile Ser Cys Thr Gly Lys His Arg His Thr
145                 150                 155                 160

Val Asn Trp Tyr Gln Leu Leu Pro Gly Thr Ala Pro Lys Leu Leu Ile
                165                 170                 175

Tyr Leu Asp Ser Glu Arg Pro Ser Gly Val Pro Asp Arg Phe Ser Gly
            180                 185                 190

Ser Lys Ser Gly Thr Ser Ala Ser Leu Ala Ile Ser Gly Leu Arg Ser
        195                 200                 205

Glu Asp Glu Ala Asp Tyr Tyr Cys Gln Ser Tyr Asp Ser Ser Ser Val
    210                 215                 220

Val Phe Gly Gly Gly Thr Lys Leu Thr Val Leu
225                 230                 235

<210>    9
<211>    717
<212>    DNA
<213>    Artificial Sequence

<220>
<223>    CL5

<400>    9
cgagtgcagc tgctggaaag tggaggtgga ctggtgcagc ctggcggcag cctgcgcctg      60

agctgtgccg ccagcggatt caccttcagc agctatgcca tgcattgggt tcgccaagca     120

cctggcaaag gcctggaatg ggtgagcagc atcagcggcg gcggctatgg cacctattat     180

gccgatagcg tgaaaggccg ctttaccatc agccgcgata acagcaaaaa caccctgtat     240

ctgcagatga acagcctgcg cgccgaggac accgcagtct actactgtgc ccgcagcacc     300

gtgtgggcct ttgatatctg gggacaaggt actctggtga ccgtgagcag cggtggagga     360

ggtagcggag gtggtggatc tggaggtgga ggtagtcaga gcgtgctgac ccagcctcct     420

agcgcctccg gtacaccagg acagcgcgtg actattagct gtagcggcaa caacatcggc     480

agcaaaagcg tgcattggta ccagcaactg cctggaactg cacctaagct gctgatctat     540

gatgtgagca aacgccctag cgagcgtcct gatcgcttta gcggtagcaa atcaggcacc     600

agcgccagtc tggccatcag cgaccttcgc tccgaagatg aagccgatta ttattgtcag     660

agctttgata gcagcggcac ctggatcttt ggtggcggta ccaagctgac cgtgctg        717

<210>    10
<211>    239
<212>    PRT
<213>    Artificial Sequence

<220>
<223>    CL5

<400>    10
Arg Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ser Ser Ile Ser Gly Gly Gly Tyr Gly Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Thr Val Trp Ala Phe Asp Ile Trp Gly Gln Gly Thr Leu
            100                 105                 110

Val Thr Val Ser Ser Gly Gly Gly Gly Ser Gly Gly Gly Gly Ser Gly
        115                 120                 125

Gly Gly Gly Ser Gln Ser Val Leu Thr Gln Pro Pro Ser Ala Ser Gly
    130                 135                 140

Thr Pro Gly Gln Arg Val Thr Ile Ser Cys Ser Gly Asn Asn Ile Gly
145                 150                 155                 160

Ser Lys Ser Val His Trp Tyr Gln Gln Leu Pro Gly Thr Ala Pro Lys
                165                 170                 175

Leu Leu Ile Tyr Asp Val Ser Lys Arg Pro Ser Glu Arg Pro Asp Arg
            180                 185                 190

Phe Ser Gly Ser Lys Ser Gly Thr Ser Ala Ser Leu Ala Ile Ser Asp
        195                 200                 205

Leu Arg Ser Glu Asp Glu Ala Asp Tyr Tyr Cys Gln Ser Phe Asp Ser
    210                 215                 220

Ser Gly Thr Trp Ile Phe Gly Gly Gly Thr Lys Leu Thr Val Leu
225                 230                 235

<210>    11
<211>    729
<212>    DNA
<213>    Artificial Sequence

<220>
<223>    CL6

<400>    11
gaggtgcagc tgttggagtc tggtggaggc ttggtacagc ctggaggttc tcttcgcctc      60

tcctgtgcag cctccggatt cactttcagc agctacggta tgcattgggt cagacaggca     120

ccaggtaagg gactggagtg ggtctctgca attagcggta gcggtggtag cacttactac     180

gcagacagcg tgaagggtcg cttcaccatc tcacgcgaca actccaagaa caccctgtac     240

ctgcagatga acagccttcg cgcagaggac actgccgtgt attactgcgc agtcagtggt     300

gcaggtcgtg gtttcttcga ctactgggga caaggtactc tggtcactgt ctcctcaggt     360

ggaggcggtt caggcggagg tggatctggc ggtggcggat cccagtctgt gctgactcag     420

ccaccttcag catctggtac tccaggtcag cgcgtcacca tcagctgcag cggtagcagc     480

agcaacattg gtagcaacta cgtgtactgg tatcagcaac tcccaggcac cgctcctaag     540

ctcctgattt acgaggacaa caagcgtcct agtggtgtgc ctgatcgctt ttctgggtcc 600

aagtctggca cctcagcctc tctggctatc agtggacttc gctccgagga cgaggctgac 660

tattactgca gcagctacac tagcagcagc actgtgatct tcggcggtgg gaccaaactg 720

accgtccta 729

<210> 12
<211> 243
<212> PRT
<213> Artificial Sequence

<220>
<223> CL6

<400> 12
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Val Ser Gly Ala Gly Arg Gly Phe Phe Asp Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Gln Ser Val Leu Thr Gln Pro Pro Ser Ala
    130                 135                 140

Ser Gly Thr Pro Gly Gln Arg Val Thr Ile Ser Cys Ser Gly Ser Ser
145                 150                 155                 160

Ser Asn Ile Gly Ser Asn Tyr Val Tyr Trp Tyr Gln Gln Leu Pro Gly
                165                 170                 175

Thr Ala Pro Lys Leu Leu Ile Tyr Glu Asp Asn Lys Arg Pro Ser Gly
            180                 185                 190

Val Pro Asp Arg Phe Ser Gly Ser Lys Ser Gly Thr Ser Ala Ser Leu
        195                 200                 205

Ala Ile Ser Gly Leu Arg Ser Glu Asp Glu Ala Asp Tyr Tyr Cys Ser
    210                 215                 220

Ser Tyr Thr Ser Ser Ser Thr Val Ile Phe Gly Gly Gly Thr Lys Leu
225                 230                 235                 240

Thr Val Leu

<210> 13
<211> 720
<212> DNA
<213> Artificial Sequence

<220>
<223> CL7

<400> 13
gaggtgcagc tgttggagtc tggtggaggc ttggtacagc ctggaggttc tcttcgcctc 60

tcctgtgcag cctccggatt cactttcagc cgctacgcaa tgagctgggt cagacaggca 120

ccaggtaagg gactggagtg ggtctctgca attagcggta gcggtggtag cacttactac 180

gcagacagcg tgaagggtcg cttcaccatc tcacgcgaca actccaagaa caccctgtac 240

ctgcagatga acagccttcg cgcagaggac actgccgtgt attactgcgc acgtagcagc 300

cagggtatct tcgacatctg gggacaaggt actctggtca ctgtctcctc aggtggaggc 360

ggttcaggcg gaggtggatc tggcggtggc ggatcccagt ctgtgctgac tcagccacct 420

tcagcatctg gtactccagg tcagcgcgtc accatcagct gcagtggtaa caatatcggt 480

actagacgcg tgcattggta tcagcaactc ccagacaccg ctcctaagct cctgatttac 540

agtaagaaca accgtcctag tggtgtgcct gatcgctttt ctgggtccaa gtctggcacc 600

tcagcctctc tggctatcag tggacttcgc tccgaggacg aggctgacta ttactgcgca 660

gcatgggacg acagcctgag cggtcctgtg ttcggcggtg ggaccaaact gaccgtccta 720

720

<210> 14
<211> 240
<212> PRT
<213> Artificial Sequence

<220>
<223> CL7

<400> 14
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Arg Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Ser Gln Gly Ile Phe Asp Ile Trp Gly Gln Gly Thr Leu
            100                 105                 110

Val Thr Val Ser Ser Gly Gly Gly Gly Ser Gly Gly Gly Gly Ser Gly
        115                 120                 125

Gly Gly Gly Ser Gln Ser Val Leu Thr Gln Pro Pro Ser Ala Ser Gly
    130                 135                 140

Thr Pro Gly Gln Arg Val Thr Ile Ser Cys Ser Gly Asn Asn Ile Gly
145                 150                 155                 160

Thr Arg Arg Val His Trp Tyr Gln Gln Leu Pro Asp Thr Ala Pro Lys
                165                 170                 175

Leu Leu Ile Tyr Ser Lys Asn Asn Arg Pro Ser Gly Val Pro Asp Arg
            180                 185                 190

Phe Ser Gly Ser Lys Ser Gly Thr Ser Ala Ser Leu Ala Ile Ser Gly
        195                 200                 205

Leu Arg Ser Glu Asp Glu Ala Asp Tyr Tyr Cys Ala Ala Trp Asp Asp
    210                 215                 220

Ser Leu Ser Gly Pro Val Phe Gly Gly Gly Thr Lys Leu Thr Val Leu
225                 230                 235                 240


<210>   15
<211>   726
<212>   DNA
<213>   Artificial Sequence

<220>
<223>   CL8


<400>   15
gaggtgcagc tgttggagtc tggtggaggc ttggtacagc ctggaggttc tcttcgcctc      60

tcctgtgcag cctccggatt cactttcagc agctacgcaa tgagctgggt cagacaggca     120

ccaggtaagg gactggagtg ggtctctgca attagcggta gcggtggtag cacttactac     180

gcagacagcg tgaagggtcg cttcaccatc tcacgcaaca actccaagaa caccctgtac     240

ctgcagatga acagccttcg cgcagaggac actgccgtgt attactgcgc acgtagcggt     300

cgttacgcag acttgacatc tgggggacaa ggtactctgg tcactgtctc ctcaggtgga     360

ggcggttcag gcggaggtgg atctggcggt ggcggatccc agtctgtgct gactcagcca     420

ccttcagcat ctggtactcc aggtcagcgc gtcaccatca gctgcagcgg tagcaacagc     480

aacatcggta acaactacgt gagctggtat cagcaactcc cagacacccc tcctaagctc     540

ctgatttacg acaacaacaa gcgtcctagt ggtgtgcctg atcgcttttc tgggtccaag 600

tctggcacct cagcctctct ggctatcagt ggacttcgct ccgaggacga ggctgactat 660

tactgcagca gctacactag cagcagcact gtgatgttcg gcggtgggac caaactgacc 720

gtccta 726

<210> 16
<211> 242
<212> PRT
<213> Artificial Sequence

<220>
<223> CL8

<400> 16
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asn Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Gly Arg Tyr Ala Asp Leu Thr Ser Gly Gly Gln Gly Thr
            100                 105                 110

Leu Val Thr Val Ser Ser Gly Gly Gly Gly Ser Gly Gly Gly Gly Ser
        115                 120                 125

Gly Gly Gly Gly Ser Gln Ser Val Leu Thr Gln Pro Pro Ser Ala Ser
    130                 135                 140

Gly Thr Pro Gly Gln Arg Val Thr Ile Ser Cys Ser Gly Ser Asn Ser
145                 150                 155                 160

Asn Ile Gly Asn Asn Tyr Val Ser Trp Tyr Gln Gln Leu Pro Asp Thr
                165                 170                 175

Pro Pro Lys Leu Leu Ile Tyr Asp Asn Asn Lys Arg Pro Ser Gly Val
            180                 185                 190

Pro Asp Arg Phe Ser Gly Ser Lys Ser Gly Thr Ser Ala Ser Leu Ala
        195                 200                 205

Ile Ser Gly Leu Arg Ser Glu Asp Glu Ala Asp Tyr Tyr Cys Ser Ser
    210                 215                 220

Tyr Thr Ser Ser Ser Thr Val Met Phe Gly Gly Gly Thr Lys Leu Thr
225                 230                 235                 240

Val Leu

<210> 17
<211> 723
<212> DNA
<213> Artificial Sequence

<220>
<223> CL9

<400> 17
gaggtgcagc tgttggagtc tggtggaggc ttggtacagc ctggaggttc tcttcgcctc 60

tcctgtgcag cctccggatt cactttcagc agctactact ggagctgggt cagacaggca 120

ccaggtaagg gactggagtg ggtctctgca attagcggta gcggtggtag cacttactac 180

gcagacagcg tgaagggtcg cttcaccatc tcacgcgaca actccaagaa caccctgtac 240

ctgcagatga acagccttcg cgcagaggac actgccgtgt attactgcgc acgtatcgac 300

gtgtacggtt tcgacatctg gggacaaggt actctggtca ctgtctcctc aggtggaggc 360

ggttcaggcg gaggtggatc tggcggtggc ggatcccagt ctgtgctgac tcagccacct 420

tcagcatctg gtactccagg tcagcgcgtc accatcagct gcagcggtag cactagcaac 480

atcggtacta actacgtgta ctggtatcag caactcccag gcaccgctcc taagctcctg 540

atttacgaca acaacaaccg tcctagtggt gtgcctgatc gcttttctgg gtccaagtct 600

ggcacctcag cctctctggc tatcagtgga cttcgctccg aggacgaggc tgactattac 660

tgccagactt gggacagcag cactgacgta gtgttcggcg gtgggaccaa actgaccgtc 720

cta 723

<210> 18
<211> 241
<212> PRT
<213> Artificial Sequence

<220>
<223> CL9

<400> 18
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Tyr Trp Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ile Asp Val Tyr Gly Phe Asp Ile Trp Gly Gln Gly Thr Leu
            100                 105                 110

Val Thr Val Ser Ser Gly Gly Gly Gly Ser Gly Gly Gly Gly Ser Gly
        115                 120                 125

Gly Gly Gly Ser Gln Ser Val Leu Thr Gln Pro Pro Ser Ala Ser Gly
    130                 135                 140

Thr Pro Gly Gln Arg Val Thr Ile Ser Cys Ser Gly Ser Thr Ser Asn
145                 150                 155                 160

Ile Gly Thr Asn Tyr Val Tyr Trp Tyr Gln Gln Leu Pro Gly Thr Ala
                165                 170                 175

Pro Lys Leu Leu Ile Tyr Asp Asn Asn Asn Arg Pro Ser Gly Val Pro
            180                 185                 190

Asp Arg Phe Ser Gly Ser Lys Ser Gly Thr Ser Ala Ser Leu Ala Ile
        195                 200                 205

Ser Gly Leu Arg Ser Glu Asp Glu Ala Asp Tyr Tyr Cys Gln Thr Trp
    210                 215                 220

Asp Ser Ser Thr Asp Val Val Phe Gly Gly Gly Thr Lys Leu Thr Val
225                 230                 235                 240

Leu

<210>    19
<211>    720
<212>    DNA
<213>    Artificial Sequence

<220>
<223>    CL10

<400>    19
gaggtgcagc tgttggagtc tggtggaggc ttggtacagc ctggaggttc tcttcgcctc      60

tcctgtgcag cctccggatt cactttcagc agctacggta tgcattgggt cagacaggca     120

ccaggtaagg gactggagtg ggtctctgca attagcggta gcggtggtag cacttactac     180

gcagacagcg tgaagggtcg cttcaccatc tcacgcgaca actccaagaa caccctgtac     240

ctgcagatga acagccttcg cgcagaggac actgccgtgt attactgcgc aagcggttac     300

ggtctgatgg acgtgtgggg acaaggtact ctggtcactg tctcctcagg tggaggcggt     360

tcaggcggag gtggatctgg cggtggcgga tcccagtctg tgctgactca gccaccttca     420

gcatctggta ctccaggtca gcgcgtcacc atcagctgca ctcgtagcag cggtatcatc     480

gcaagcaact acgtgcagtg gtatcagcaa ctcccaggca ccgctcctaa gctcctgatt 540

taccgcaaca accagcgccc tagtggtgtg cctgatcgct tttctgggtc caagtctggc 600

acctcagcct ctctggctat cagtggactt cgctccgagg acgaggctga ctattactgc 660

agcagctacg caggtaacaa caacctggtg ttcggcggtg ggaccaaact gaccgtccta 720

720

<210> 20
<211> 240
<212> PRT
<213> Artificial Sequence

<220>
<223> CL10

<400> 20
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ser Gly Tyr Gly Leu Met Asp Val Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Gln Ser Val Leu Thr Gln Pro Pro Ser Ala Ser Gly Thr
    130                 135                 140

Pro Gly Gln Arg Val Thr Ile Ser Cys Thr Arg Ser Ser Gly Ile Ile
145                 150                 155                 160

Ala Ser Asn Tyr Val Gln Trp Tyr Gln Gln Leu Pro Gly Thr Ala Pro
                165                 170                 175

Lys Leu Leu Ile Tyr Arg Asn Asn Gln Arg Pro Ser Gly Val Pro Asp
            180                 185                 190

Arg Phe Ser Gly Ser Lys Ser Gly Thr Ser Ala Ser Leu Ala Ile Ser
        195                 200                 205

Gly Leu Arg Ser Glu Asp Glu Ala Asp Tyr Tyr Cys Ser Ser Tyr Ala
    210                 215                 220

Gly Asn Asn Asn Leu Val Phe Gly Gly Gly Thr Lys Leu Thr Val Leu
225                 230                 235                 240

<210>    21
<211>    717
<212>    DNA
<213>    Artificial Sequence

<220>
<223>    SK11

<400>    21
ggccgagtgc agctgctgga aagtggaggt ggactggtgc agcctggcgg cagcctgcgc      60

ctgagctgtg ccgccagcgg attcaccttc agcacctatg gcatgcattg ggttcgccaa     120

gcacctggca aaggcctgga atgggtgagc gccatcagcg gcagcggcgg cagcacctat     180

tatgccgata gcgtgaaagg ccgctttacc atcagccgcg ataacagcaa aaacaccctg     240

tatctgcaga tgaacagcct gcgcgccgag gacaccgcag tctactactg tgcccgcggc     300

ctgagcggcc ttgattattg gggacaaggt actctggtga ccgtgagcag cagtggagga     360

ggtagcggag gtagtggatc tggaggtgga ggtagtgaaa tcgtgctgac ccagagccct     420

ggcaccctga gcctgagccc tggcgaacgc gcaacactgt catgccgctc cagccagggc     480

atcaccaact atctggcctg gtatcagcag aaaccaggtc aggctccacg tctgctgatc     540

tatgatgcca gcaaccgcgc caccggcatc cctgatcgct tctcaggatc tggaagcggt     600

accgatttta ccctgaccat cagccgcctg gaacctgagg actttgccgt gtattattgt     660

cagcagagct atagcacccc tctgaccttc ggtcagggca ctaaagtgga aatcaaa        717

<210>    22
<211>    238
<212>    PRT
<213>    Artificial Sequence

<220>
<223>    SK11

<400>    22
Arg Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Gly Met His Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr

65                  70                  75                  80

Leu Gln Met Asn Ser Leu Arg Ala Glu Asp Thr Ala Val Tyr Tyr Cys
                85                  90                  95

Ala Arg Gly Leu Ser Gly Leu Asp Tyr Trp Gly Gln Gly Thr Leu Val
            100                 105                 110

Thr Val Ser Ser Ser Gly Gly Gly Ser Gly Gly Ser Gly Ser Gly Gly
        115                 120                 125

Gly Gly Ser Glu Ile Val Leu Thr Gln Ser Pro Gly Thr Leu Ser Leu
    130                 135                 140

Ser Pro Gly Glu Arg Ala Thr Leu Ser Cys Arg Ser Ser Gln Gly Ile
145                 150                 155                 160

Thr Asn Tyr Leu Ala Trp Tyr Gln Gln Lys Pro Gly Gln Ala Pro Arg
                165                 170                 175

Leu Leu Ile Tyr Asp Ala Ser Asn Arg Ala Thr Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Ser Tyr Ser
    210                 215                 220

Thr Pro Leu Thr Phe Gly Gln Gly Thr Lys Val Glu Ile Lys
225                 230                 235

<210>    23
<211>    717
<212>    DNA
<213>    Artificial Sequence

<220>
<223>    SK12

<400>    23
ggccgagtgc agctgctgga aagtggaggt ggactggtgc agcctggcgg cagcctgcgc      60

ctgagctgtg ccgccagcgg attcaccttc agcagctatg ccatgcattg ggttcgccaa     120

gcacctggca aaggcctgga atgggtgagc gccatcagcg gcagcggcgg cgatacctat     180

catgccgata gcgtgaaagg ccgctttacc atcagccgcg ataacagcaa aaacaccctg     240

tatctgcaga tgaacagcct gcgcgccgag gacaccgcag tctactactg tacccgcggc     300

ctgagcggct ttgattattg gggacaaggt actctggtga ccgtgagcag cggtggagga     360

ggtagcggag gtggtggatc tggaggtgga ggtagtgaaa tcgtgctgac ccagagccct     420

ggcaccctga gcctgagccc tggcgaacgc gcaacactgt catgccgcgc cagccagagc     480

atcagcagct atctgaactg gtatcagcag aaaccaggtc aggctccacg tctgctgatc     540

tatgatgcca gcaaccgcgc ccctggcatc cctgatcgct tctcaggatc tggaagcggt     600

accgatttta ccctgaccat cagccgcctg gaacctgagg actttgccgt gtattattgt 660

cagcagagct atagcatccc tatcaccttc ggtcagggca ctaaagtgga aatcaaa 717

<210> 24
<211> 238
<212> PRT
<213> Artificial Sequence

<220>
<223> SK12

<400> 24
Arg Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ser Ala Ile Ser Gly Ser Gly Gly Asp Thr Tyr His Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Arg Gly Leu Ser Gly Phe Asp Tyr Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Glu Ile Val Leu Thr Gln Ser Pro Gly Thr Leu Ser Leu
    130                 135                 140

Ser Pro Gly Glu Arg Ala Thr Leu Ser Cys Arg Ala Ser Gln Ser Ile
145                 150                 155                 160

Ser Ser Tyr Leu Asn Trp Tyr Gln Gln Lys Pro Gly Gln Ala Pro Arg
                165                 170                 175

Leu Leu Ile Tyr Asp Ala Ser Asn Arg Ala Pro Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Ser Tyr Ser
    210                 215                 220

Ile Pro Ile Thr Phe Gly Gln Gly Thr Lys Val Glu Ile Lys
225                 230                 235

<210> 25
<211> 717

<212>    DNA
<213>    Artificial Sequence

<220>
<223>    SK13


<400>    25
ggccgagtgc agctgctgga aagtggaggt ggactggtgc agcctggcgg cagcctgcgc      60

ctgagctgtg ccgccagcgg attcaccttc agcgattatg ccatgagctg ggttcgccaa     120

gcacctggca aaggcctgga atgggtgagc agcatcagca gcagcagcag ctatatctac     180

tataccgata gcgtgaaagg ccgctttacc atcagccgcg ataacagcaa aaacaccctg     240

tatctgcaga tgaacagcct gcgcgccgag gacaccgcag tctactactg tgcccgcggc     300

ggctatggct ttgattattg gggacaaggt accctggtga ccgtgagcag cggtggagga     360

ggtagcggag gtggtggatc tggaggtgga ggtagtgaaa tcgtgctgac ccagagccct     420

ggcaccctga gcctgagccc tggcgaacgc gcaacactgt catgccgcgc cagccagagc     480

atcagcagct atctgaactg gtatcagcag aaaccaggtc aggctccacg tctgctgatc     540

tatagcgcca gcagccgccc acagggcatc cccgatcgct tctcaggatc tggaagcggt     600

accgatttta ccctgaccat cagccgcctg gaacctgagg actttgccgt gtattattgt     660

cagcagtatg atgatctgcc ttttaccttc ggtcagggca ctaaagtgga aatcaaa        717


<210>    26
<211>    238
<212>    PRT
<213>    Artificial Sequence

<220>
<223>    SK13


<400>    26
Arg Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Ala Met Ser Trp Val Arg Gln Ala Pro Gly Lys Gly Leu Glu Trp Val
        35                  40                  45

Ser Ser Ile Ser Ser Ser Ser Ser Tyr Ile Tyr Tyr Thr Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Gly Tyr Gly Phe Asp Tyr Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Glu Ile Val Leu Thr Gln Ser Pro Gly Thr Leu Ser Leu
    130                 135                 140

Ser Pro Gly Glu Arg Ala Thr Leu Ser Cys Arg Ala Ser Gln Ser Ile
145                 150                 155                 160

Ser Ser Tyr Leu Asn Trp Tyr Gln Gln Lys Pro Gly Gln Ala Pro Arg
                165                 170                 175

Leu Leu Ile Tyr Ser Ala Ser Ser Arg Pro Gln Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Tyr Asp Asp
    210                 215                 220

Leu Pro Phe Thr Phe Gly Gln Gly Thr Lys Val Glu Ile Lys
225                 230                 235

<210>    27
<211>    714
<212>    DNA
<213>    Artificial Sequence

<220>
<223>    SK14

<400>    27
gaagtgcagc tgctggaaag tggaggtgga ctggtgcagc ctggcggcag cctgcgcctg      60

agctgtgccg ccagcggatt caccttcagc aactttgcga tcgcctgggt tcgccaagca     120

cctggcaaag gcctggaatg ggtgagcgcc atcagcggcc gcggcaccag cacctattat     180

gccgatagcg tgaaaggccg ctttaccatc agccgcgata acagcaaaaa caccctgtat     240

ctgcagatga acagcctgcg cgccgaggac accgcagtct actactgtgc ccgcggcgtg     300

agcggctttg atagctgggg acaaggtact ctggtgaccg tgagcagcgg tggaggaggt     360

agcggaggtg gtggatctgg aggtggaggt agtgaaatcg tgctgaccca gagccctggc     420

accctgagcc tgagccctgg cgaacgcgca acactgtcat gccgcgccag ccagagcatc     480

agcagccatc tggcctggta tcagcagaaa ccaggtcagg ctccacgtct gctgatctat     540

gataccagca accgcgccac cggcatccct gatcgcttct caggatctgg gagcggtacc     600

gattttaccc tgaccatcag ccgcctggaa cctgaggact ttgccgtgta ctattgtcag     660

cagagctata gcaccccttt taccttcggt cagggcacta aagtggaaat caaa           714

<210>    28
<211>    238

<212>	PRT
<213>	Artificial Sequence

<220>
<223>	SK14


<400>	28
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Phe
            20                  25                  30

Ala Ile Ala Trp Val Arg Gln Ala Pro Gly Lys Gly Leu Glu Trp Val
        35                  40                  45

Ser Ala Ile Ser Gly Arg Gly Thr Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Val Ser Gly Phe Asp Ser Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Glu Ile Val Leu Thr Gln Ser Pro Gly Thr Leu Ser Leu
    130                 135                 140

Ser Pro Gly Glu Arg Ala Thr Leu Ser Cys Arg Ala Ser Gln Ser Ile
145                 150                 155                 160

Ser Ser His Leu Ala Trp Tyr Gln Gln Lys Pro Gly Gln Ala Pro Arg
                165                 170                 175

Leu Leu Ile Tyr Asp Thr Ser Asn Arg Ala Thr Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Ser Tyr Ser
    210                 215                 220

Thr Pro Phe Thr Phe Gly Gln Gly Thr Lys Val Glu Ile Lys
225                 230                 235


<210>	29
<211>	717
<212>	DNA
<213>	Artificial Sequence

<220>
<223>	SK15

<400>    29
ggccgagtgc agctgctgga aagtggaggt ggactggtgc agcctggcgg cagcctgcgc    60

ctgagctgtg ccgccagcgg attcaccttc agcagctatg ccatgcattg ggttcgccaa    120

gcacctggca aaggcctgga atgggtgagc gccatcaacg gcagcggcgg cagcacctat    180

tatgccgata gcgtgaaagg ccgctttacc atcagccgcg ataacagcaa aaacaccctg    240

tatctgcaga cgaacagcct gcgcgccgag gacaccgcag tctactactg tgcccgcggc    300

ctgcagggct ttgattattg gggacaaggt actctggtga ccgtgagcag cagtggagga    360

ggtagcggag gtggtggatc tggaggtgga ggtagtgaaa tcgtgctgac ccagagccct    420

ggcaccctga gcctgagccc tggcgaacgc gcaacactgt catgccaggc cagccaggat    480

atcaccaact atctgaactg gtatcagcag aaaccaggtc aggctccacg tctgctgatc    540

tatgatgcca gcagcctgga aaccggcatc cctgatcgtt tctcaggatc tggaagcggt    600

accgatttta ccctgaccat cagccgcctg gaacctgagg actttgccgt gtattattgt    660

cagcagagct atagcacccc tatcaccttc ggtcagggca ctaaagtgga aatcaaa    717


<210>    30
<211>    238
<212>    PRT
<213>    Artificial Sequence

<220>
<223>    SK15


<400>    30
Arg Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ser Ala Ile Asn Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Thr Asn Ser Leu Arg Ala Glu Asp Thr Ala Val Tyr Tyr Cys
                85                  90                  95

Ala Arg Gly Leu Gln Gly Phe Asp Tyr Trp Gly Gln Gly Thr Leu Val
            100                 105                 110

Thr Val Ser Ser Ser Gly Gly Gly Ser Gly Gly Gly Gly Ser Gly Gly
        115                 120                 125

Gly Gly Ser Glu Ile Val Leu Thr Gln Ser Pro Gly Thr Leu Ser Leu
    130                 135                 140

Ser Pro Gly Glu Arg Ala Thr Leu Ser Cys Gln Ala Ser Gln Asp Ile
145                 150                 155                 160

Thr Asn Tyr Leu Asn Trp Tyr Gln Gln Lys Pro Gly Gln Ala Pro Arg
                165                 170                 175

Leu Leu Ile Tyr Asp Ala Ser Ser Leu Glu Thr Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Ser Tyr Ser
    210                 215                 220

Thr Pro Ile Thr Phe Gly Gln Gly Thr Lys Val Glu Ile Lys
225                 230                 235


<210>    31
<211>    717
<212>    DNA
<213>    Artificial Sequence

<220>
<223>    SK16


<400>    31
ggccgagtgc agctgctgga aagtggaggt ggactggtgc agcctggcgg cagcctgcgc      60

ctgagctgtg ccgccagcgg attcaccttc agcagctatg ccatgagctg ggttcgccaa     120

gcacctggca aaggcctgga atgggtgagc gccatcaacg gcagcggcgg cagcaccctg     180

tatgccgata gcgtgaaagg ccgctttacc atcagccgcg ataacagcaa aaacaccctg     240

tatctgcaga tgaacagcct gcgcgccgag gacaccgcag tctactactg tgcccgcggc     300

gtgagcggct ttgatagctg gggacaaggt actctggtga ccgtgagcag cggtggagga     360

ggtagcggag gtggtggatc tggaggtgga ggtagtgaaa tcgtgctgac ccagagccct     420

ggcaccctga gcctgagccc tggcgaacgc gcaacactgt catgccgcat cagccagagc     480

atcagcagct atctgaactg gtatcagcag aaaccaggtc aggctccacg tctgctgatc     540

tatgatgcca gcctgcgcgc caccggcatc cctgatcgct tctcaggatc tggaagcggt     600

accgatttta ccctgaccat cagccgcctg gaacctgagg actttgccgt gtattattgt     660

cagcagagct ataaaacccc tatcaccttc ggtcagggca ctaaagtgga aatcaaa        717


<210>    32
<211>    238
<212>    PRT
<213>    Artificial Sequence

<220>
<223>    SK16

<400>    32
Arg Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Asn Gly Ser Gly Gly Ser Thr Leu Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Val Ser Gly Phe Asp Ser Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Glu Ile Val Leu Thr Gln Ser Pro Gly Thr Leu Ser Leu
    130                 135                 140

Ser Pro Gly Glu Arg Ala Thr Leu Ser Cys Arg Ile Ser Gln Ser Ile
145                 150                 155                 160

Ser Ser Tyr Leu Asn Trp Tyr Gln Gln Lys Pro Gly Gln Ala Pro Arg
                165                 170                 175

Leu Leu Ile Tyr Asp Ala Ser Leu Arg Ala Thr Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Ser Tyr Lys
    210                 215                 220

Thr Pro Ile Thr Phe Gly Gln Gly Thr Lys Val Glu Ile Lys
225                 230                 235


<210>    33
<211>    714
<212>    DNA
<213>    Artificial Sequence

<220>
<223>    SK17


<400>    33
gaagtgcagc tgctggaaag tggaggtgga ctggtgcagc ctggcggcag cctgcgcctg      60

agctgtgccg ccagcggatt caccttcagc agctattatt ggagctgggt tcgccaagca     120

cctggcaaag gcctggaatg ggtgagcacc atcaccggca gcggcggcag caccgattat     180

gccaacagcg tgaaaggccg ctttaccatc agccgcgata acagcaaaaa caccctgtat 240

ctgcagatga acagcctgcg cgccgaggac accgcagtct actactgtgc caccggcggc 300

ggcatctttg actattgggg acaaggtact ctggtgaccg tgagcagcgg tggaggaggt 360

agcggaggtg gtggatctgg aggtggaggt agtgaaatcg tgctgaccca gagccctggc 420

accctgagcc tgagccctgg cgaacgcgca acactgtcat gccaggccag ccagaccatc 480

agcaactatc tgaactggta tcagcagaaa ccaggtcagg ctccacgtct gctgatctat 540

gatgccagca accgcgccac cggcatccct gatcgcttct caggatctgg aagcggtacc 600

gattttaccc tgaccatcag ccgcctggaa cctgaggact ttgccgtgta ttattgtcag 660

cagtacaaca gctatcctcc tagcttcggt cagggcacta aagtggaaat caaa 714

<210> 34
<211> 238
<212> PRT
<213> Artificial Sequence

<220>
<223> SK17

<400> 34
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Tyr Trp Ser Trp Val Arg Gln Ala Pro Gly Lys Gly Leu Glu Trp Val
        35                  40                  45

Ser Thr Ile Thr Gly Ser Gly Gly Ser Thr Asp Tyr Ala Asn Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Gly Gly Gly Ile Phe Asp Tyr Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Glu Ile Val Leu Thr Gln Ser Pro Gly Thr Leu Ser Leu
    130                 135                 140

Ser Pro Gly Glu Arg Ala Thr Leu Ser Cys Gln Ala Ser Gln Thr Ile
145                 150                 155                 160

Ser Asn Tyr Leu Asn Trp Tyr Gln Gln Lys Pro Gly Gln Ala Pro Arg
                165                 170                 175

Leu Leu Ile Tyr Asp Ala Ser Asn Arg Ala Thr Gly Ile Pro Asp Arg

180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Tyr Asn Ser
    210                 215                 220

Tyr Pro Pro Ser Phe Gly Gln Gly Thr Lys Val Glu Ile Lys
225                 230                 235

<210>    35
<211>    717
<212>    DNA
<213>    Artificial Sequence

<220>
<223>    SL18

<400>    35
cgagtgcagc tgctggaaag tggaggtgga ctggtgcagc ctggcggcag cctgcgcctg      60

agctgtgccg ccagcggatt caccttcagc gattatcata tgcattgggt tcgccaagca     120

cctggcaaag gcctggaatg ggtgagcacc atcagcagca gcggcggcta tacctattat     180

gccgaaagcg tgaaaagccg ctttaccatc agccgcgata acagcaaaaa caccctgtat     240

ctgcagatga acagcctgcg cgccgaggac accgcagtct actactgtgc ccgatcgata     300

cgcctgcctc tggattattg gggacaaggt actctggtga ccgtgagcag cagtggagga     360

ggtagcggag gtggtggatc tggaggtgga ggtagtcaga gcgtgctgac ccagcctcct     420

agcgcctccg gtacaccagg acagcgcgtg actattagct gtagcggcaa caacatcggc     480

agcaaaggcg tgcattggta tcagcaactg cctggaactg cacctaagct gctgatctat     540

gaagatagca aacgccctag cggcgtgcgt gatcgcttta gcggtagcaa atcaggcacc     600

agcgccagcc tggccatcag cggccttcgc tccgaagatg aagccgatta ttattgtcag     660

agctatgata gcaccaaagg cgtggtgttt ggtggcggta ccaagctgac cgtgctg        717

<210>    36
<211>    239
<212>    PRT
<213>    Artificial Sequence

<220>
<223>    SL18

<400>    36
Arg Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

His Met His Trp Val Arg Gln Ala Pro Gly Lys Gly Leu Glu Trp Val
        35                  40                  45

Ser Thr Ile Ser Ser Ser Gly Gly Tyr Thr Tyr Tyr Ala Glu Ser Val
    50                  55                  60

Lys Ser Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Ile Arg Leu Pro Leu Asp Tyr Trp Gly Gln Gly Thr Leu
            100                 105                 110

Val Thr Val Ser Ser Ser Gly Gly Gly Ser Gly Gly Gly Gly Ser Gly
        115                 120                 125

Gly Gly Gly Ser Gln Ser Val Leu Thr Gln Pro Pro Ser Ala Ser Gly
    130                 135                 140

Thr Pro Gly Gln Arg Val Thr Ile Ser Cys Ser Gly Asn Asn Ile Gly
145                 150                 155                 160

Ser Lys Gly Val His Trp Tyr Gln Gln Leu Pro Gly Thr Ala Pro Lys
                165                 170                 175

Leu Leu Ile Tyr Glu Asp Ser Lys Arg Pro Ser Gly Val Arg Asp Arg
            180                 185                 190

Phe Ser Gly Ser Lys Ser Gly Thr Ser Ala Ser Leu Ala Ile Ser Gly
        195                 200                 205

Leu Arg Ser Glu Asp Glu Ala Asp Tyr Tyr Cys Gln Ser Tyr Asp Ser
    210                 215                 220

Thr Lys Gly Val Val Phe Gly Gly Gly Thr Lys Leu Thr Val Leu
225                 230                 235


<210>    37
<211>    720
<212>    DNA
<213>    Artificial Sequence

<220>
<223>    CB301_H3L1_A10


<400>    37
gaggtgcagc tgttggagtc tggtggaggc ttggtacagt ctggaggttc tcttcgcctc      60

tcctgtgcag cctccggatt cactttcagc agctacggta tgcattgggt cagacaggca     120

ccaggtaagg gactggagtg ggtctctgca attagcggta gcggtggtag cacttactac     180

gcagacagcg tgaagggtcg cttcaccatc tcacgcgaca actccaagaa caccctgtac     240

ctgcagatga acagccttcg cgcagaggac actgccgtgt attactgcgt gcgtggttac     300

ggtgcaatgg acgtgtgggg acaaggtact ctggtcactg tctcctcagg tggaggcggt     360

tcaggcggag gtggatctgg cggtggcgga tcccagtctg tgctgactca gccaccttca     420

gcatctggta ctccaggtca gcgcgtcacc atcagctgca ctcgtagcag cggtagcatc     480

gcaagcaact acgtgcagtg gtatcagcaa ctcccaggca ccgctcctaa gctcctgatt     540

taccgcaaca accagcgccc tagtggtgtg cctgatcgct tttctgggtc caagtctggc     600

acctcagcct ctctggctat cagtggactt cgctccgagg acgaggctga ctattactgc     660

agcagctaca ctactagcag cactctggtg ttcggcggtg ggaccaaact gaccgtccta     720

                                                                      720

<210>    38
<211>    240
<212>    PRT
<213>    Artificial Sequence

<220>
<223>    CB301_H3L1_A10

<400>    38
Glu Val Gln Leu Leu Glu Ser Gly Gly Gly Leu Val Gln Ser Gly Gly
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Val Arg Gly Tyr Gly Ala Met Asp Val Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Gln Ser Val Leu Thr Gln Pro Pro Ser Ala Ser Gly Thr
    130                 135                 140

Pro Gly Gln Arg Val Thr Ile Ser Cys Thr Arg Ser Ser Gly Ser Ile
145                 150                 155                 160

Ala Ser Asn Tyr Val Gln Trp Tyr Gln Gln Leu Pro Gly Thr Ala Pro
                165                 170                 175

Lys Leu Leu Ile Tyr Arg Asn Asn Gln Arg Pro Ser Gly Val Pro Asp
            180                 185                 190

Arg Phe Ser Gly Ser Lys Ser Gly Thr Ser Ala Ser Leu Ala Ile Ser
        195                 200                 205

Gly Leu Arg Ser Glu Asp Glu Ala Asp Tyr Tyr Cys Ser Ser Tyr Thr
    210                 215                 220

Thr Ser Ser Thr Leu Val Phe Gly Gly Gly Thr Lys Leu Thr Val Leu
225                 230                 235                 240


<210>    39
<211>    723
<212>    DNA
<213>    Artificial Sequence

<220>
<223>    CB301_H3L1_A12


<400>    39
gaggtgcagc tgttggagtc tggtggaggc ttggtacagc ctggaggttc tcttcgcctc      60

tcctgtgcag cctccggatt cactttcagc agctacgcaa tgcattgggt cagacaggca     120

ccaggtaagg gactggagtg ggtctctgca attagcggta gcggtggtag cacttactac     180

gcagacagcg tgaagggtcg cttcaccatc tcacgcgaca actccaagaa caccctgtac     240

ctgcagatga acagccttcg cgcaaaggac actgccgtgt attactgcgc aagcggctac     300

ggtctgatgg acgtatgggg acaaggtact ctggtcactg tctcctcagg tggaggcggt     360

tcaggcggag gtggatctgg cggtggcgga tcccagtctg tgctgactca gccaccttca     420

gcatctggta ctccaggtca gcgcgtcacc atcagctgca ctggtactag cagcgacgtg     480

ggtaactaca acctggtgag ctggtatcag caactcccag gcaccgctcc taagctcctg     540

atttacagca acaaccagcg ccctagtggt gtgcctgatc gcttttctgg gtccaagtct     600

ggcacctcag cctctctggc tatcagtgga cttcgctccg aggacgaggc tgactattac     660

tgcagcagct acactggtag caacgctctg ttgttcggcg gtgggaccaa actgaccgtc     720

cta                                                                   723


<210>    40
<211>    241
<212>    PRT
<213>    Artificial Sequence

<220>
<223>    CB301_H3L1_A12


<400>    40
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Ser Tyr
             20                  25                  30

Ala Met His Trp Val Arg Gln Ala Pro Gly Lys Gly Leu Glu Trp Val
         35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Lys Asp Thr Ala Val Tyr Tyr Cys
                85                  90                  95

Ala Ser Gly Tyr Gly Leu Met Asp Val Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Gln Ser Val Leu Thr Gln Pro Pro Ser Ala Ser Gly Thr
    130                 135                 140

Pro Gly Gln Arg Val Thr Ile Ser Cys Thr Gly Thr Ser Ser Asp Val
145                 150                 155                 160

Gly Asn Tyr Asn Leu Val Ser Trp Tyr Gln Gln Leu Pro Gly Thr Ala
                165                 170                 175

Pro Lys Leu Leu Ile Tyr Ser Asn Asn Gln Arg Pro Ser Gly Val Pro
            180                 185                 190

Asp Arg Phe Ser Gly Ser Lys Ser Gly Thr Ser Ala Ser Leu Ala Ile
        195                 200                 205

Ser Gly Leu Arg Ser Glu Asp Glu Ala Asp Tyr Tyr Cys Ser Ser Tyr
    210                 215                 220

Thr Gly Ser Asn Ala Leu Leu Phe Gly Gly Gly Thr Lys Leu Thr Val
225                 230                 235                 240

Leu

<210> 41
<211> 717
<212> DNA
<213> Artificial Sequence

<220>
<223> CB301_H3L1_E6

<400> 41
gaggtgcagc tgttggagtc tggtggaggc ttggtacagc ctggaggttc tcttcgcctc 60

tcctgtgcag cctccggatt cactttcagc agctacgcaa tgagctgggt cagacaggca 120

ccaggtaagg gactggagtg ggtctctgca attagcggta gcggtggtag cacttactac 180

gcagacagcg tgaagggtcg cttcaccatc tcacgcgaca actccaagaa caccctgtac 240

ctgcagatga acagccttcg cgcagaggac actgccgtgt attactgcgc acgctggcat 300

tacagcttcg actactgggg acaaggtact ctggtcactg tctcctcagg tggaggcggt 360

tcaggcggag gtggatctgg cggtggcgga tcccagtctg tgctgactca gccaccttca 420

gcatctggta ctccaggtca gcgcgtcacc atcagctgcc gtggtaacaa catcggtagc 480

aagcgtgtgc attggtatca gcaactccca ggcaccgctc ctaagctcct gatttacagc 540

tacaaccacc gtcctagcgg tgtgcctgat cgcttttctg ggtccaagtc tggcacctca 600

gcctctctgg ctatcactgg acttcgctcc gaggacgaag ctgactatta ctgcaacact 660

tgggacgaca gcctggaggg tcctgtgttc ggcggtggga ccaaactgac cgtccta 717

<210> 42
<211> 239
<212> PRT
<213> Artificial Sequence

<220>
<223> CB301_H3L1_E6

<400> 42
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Trp His Tyr Ser Phe Asp Tyr Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Gln Ser Val Leu Thr Gln Pro Pro Ser Ala Ser Gly Thr
    130                 135                 140

Pro Gly Gln Arg Val Thr Ile Ser Cys Arg Gly Asn Asn Ile Gly Ser
145                 150                 155                 160

Lys Arg Val His Trp Tyr Gln Gln Leu Pro Gly Thr Ala Pro Lys Leu
                165                 170                 175

Leu Ile Tyr Ser Tyr Asn His Arg Pro Ser Gly Val Pro Asp Arg Phe
            180                 185                 190

Ser Gly Ser Lys Ser Gly Thr Ser Ala Ser Leu Ala Ile Thr Gly Leu
        195                 200                 205

Arg Ser Glu Asp Glu Ala Asp Tyr Tyr Cys Asn Thr Trp Asp Asp Ser
    210                 215                 220

Leu Glu Gly Pro Val Phe Gly Gly Gly Thr Lys Leu Thr Val Leu
225                 230                 235

<210>    43
<211>    714
<212>    DNA
<213>    Artificial Sequence

<220>
<223>    CB301_H3L1_F4

<400>    43
gaggtgcagc tgttggagtc tggtggaggc ttggtacagc ctggaggttc tcttcgcctc        60

tcctgtgcag cctccggatt cactttcagc ggctacgcaa tgagctgggt cagacaggca       120

ccaggtaagg gactggagtg ggtctctgca attagcggta gcggtggtag cacttactac       180

gcagacagcg tgaagggtcg cttcaccatc tcacgcgaca actccaagaa caccctgtac       240

ctgcagatga acagccttcg cgcagaggac actgccgtgt attactgcgc acgtagtcct       300

agcggtctgt tcgactactg gggacaaggt actctggtca ctgtctcctc aggtggaggc       360

ggttcaggcg gaggtggatc tggcggtggc ggattccagt ctgtgctgac tcagccacct       420

tcagcatctg gtactccagg tcagcgcgtc accatcagct gcggtggtaa caacatcggt       480

agcaagcgtg tgcattggta tcagcaactc ccaggcaccg ctcctaagct cctgatttac       540

aacactagca acaagcatag cggtgtgcct gatcgctttt ctgggtccaa gtctggcacc       600

tcagcctctc tggctatcag tggacttcgc tccgaggacg aggctgacta ttactgcagc       660

agctacctac agcagcactc tctgttcggc ggtgggacca aactaaccgt ccta             714

<210>    44
<211>    238
<212>    PRT
<213>    Artificial Sequence

<220>
<223>    CB301_H3L1_F4

<400>    44
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Gly Tyr
             20                  25                  30

Ala Met Ser Trp Val Arg Gln Ala Pro Gly Lys Gly Leu Glu Trp Val
         35                  40                  45

Ser Ala Ile Ser Gly Ser Gly Gly Ser Thr Tyr Tyr Ala Asp Ser Val
     50                  55                  60

Lys Gly Arg Phe Thr Ile Ser Arg Asp Asn Ser Lys Asn Thr Leu Tyr

65                  70                  75                  80

Leu Gln Met Asn Ser Leu Arg Ala Glu Asp Thr Ala Val Tyr Tyr Cys
                85                  90                  95

Ala Arg Ser Pro Ser Gly Leu Phe Asp Tyr Trp Gly Gln Gly Thr Leu
            100                 105                 110

Val Thr Val Ser Ser Gly Gly Gly Gly Ser Gly Gly Gly Gly Ser Gly
        115                 120                 125

Gly Gly Gly Phe Gln Ser Val Leu Thr Gln Pro Pro Ser Ala Ser Gly
    130                 135                 140

Thr Pro Gly Gln Arg Val Thr Ile Ser Cys Gly Gly Asn Asn Ile Gly
145                 150                 155                 160

Ser Lys Arg Val His Trp Tyr Gln Gln Leu Pro Gly Thr Ala Pro Lys
                165                 170                 175

Leu Leu Ile Tyr Asn Thr Ser Asn Lys His Ser Gly Val Pro Asp Arg
            180                 185                 190

Phe Ser Gly Ser Lys Ser Gly Thr Ser Ala Ser Leu Ala Ile Ser Gly
        195                 200                 205

Leu Arg Ser Glu Asp Glu Ala Asp Tyr Tyr Cys Ser Ser Tyr Leu Gln
    210                 215                 220

Gln His Ser Leu Phe Gly Gly Gly Thr Lys Leu Thr Val Leu
225                 230                 235

<210>    45
<211>    729
<212>    DNA
<213>    Artificial Sequence

<220>
<223>    CB301_H3L1_G11

<400>    45
gaggtgcagc tgttggagtc tggtggaggc ttggtacagc ctggaggttc ctcttccgcc      60

tcctcctgtg cagcctccgg attcactttc agcagctacg caatgagctg ggtcagacag     120

gcaccaggta agggactgga gtgggtctct gcaattagcg gtagcggtgg tagcacttac     180

tacgcagaca gcgtgaaggg tcgcttcacc atctcacgcg acaactccaa gaacaccctg     240

tacctgcaga tgaacagcct tcgcgcagag gacactgccg tgtattactg cacacgtttc     300

gtgggtgcaa tcggtgcatt cgactactgg ggacaaggta ctctggtcac tgtctcctca     360

ggtggaggcg gttcaggcgg aggtggatct ggcggtggcg gatcccagtc tgtgctgact     420

cagccacctt cagcatctgg tactccaggt cagcgcgtca ccatcagctg cagtggtaac     480

aacatcggta gccgtagcgt gcattggtat cagcaactcc caggcaccgc tcctaagctc     540

ctgatttacc gcaacaacca gcgccctagt ggtgtgcctg atcgcttttc tgggtccaag     600

tctggcacct cagcctctct ggctatcagt ggacttcgct ccgaggacga ggctgactat 660

tactgcgcag catgggacga cagcctgagc ggtcctgtgt tcggcggtgg gaccaaactg 720

accgtccta 729

<210> 46
<211> 243
<212> PRT
<213> Artificial Sequence

<220>
<223> CB301_H3L1_G11

<400> 46
Glu Val Gln Leu Leu Glu Ser Gly Gly Gly Leu Val Gln Pro Gly Gly
1               5                   10                  15

Ser Ser Ser Ala Ser Ser Cys Ala Ala Ser Gly Phe Thr Phe Ser Ser
            20                  25                  30

Tyr Ala Met Ser Trp Val Arg Gln Ala Pro Gly Lys Gly Leu Glu Trp
        35                  40                  45

Val Ser Ala Ile Ser Gly Ser Gly Gly Ser Thr Tyr Tyr Ala Asp Ser
    50                  55                  60

Val Lys Gly Arg Phe Thr Ile Ser Arg Asp Asn Ser Lys Asn Thr Leu
65                  70                  75                  80

Tyr Leu Gln Met Asn Ser Leu Arg Ala Glu Asp Thr Ala Val Tyr Tyr
                85                  90                  95

Cys Thr Arg Phe Val Gly Ala Ile Gly Ala Phe Asp Tyr Trp Gly Gln
            100                 105                 110

Gly Thr Leu Val Thr Val Ser Ser Gly Gly Gly Gly Ser Gly Gly Gly
        115                 120                 125

Gly Ser Gly Gly Gly Gly Ser Gln Ser Val Leu Thr Gln Pro Pro Ser
    130                 135                 140

Ala Ser Gly Thr Pro Gly Gln Arg Val Thr Ile Ser Cys Ser Gly Asn
145                 150                 155                 160

Asn Ile Gly Ser Arg Ser Val His Trp Tyr Gln Gln Leu Pro Gly Thr
                165                 170                 175

Ala Pro Lys Leu Leu Ile Tyr Arg Asn Asn Gln Arg Pro Ser Gly Val
            180                 185                 190

Pro Asp Arg Phe Ser Gly Ser Lys Ser Gly Thr Ser Ala Ser Leu Ala
        195                 200                 205

Ile Ser Gly Leu Arg Ser Glu Asp Glu Ala Asp Tyr Tyr Cys Ala Ala
    210                 215                 220

Trp Asp Asp Ser Leu Ser Gly Pro Val Phe Gly Gly Gly Thr Lys Leu
225                 230                 235                 240

Thr Val Leu

<210>    47
<211>    738
<212>    DNA
<213>    Artificial Sequence

<220>
<223>    CB301_OPALTL_B5


<400>    47
gaggtgcagc tgttggagtc tggtggaggc ttggtacagc ctggaggttc tcttcgcctc      60

tcctgtgcag cctccggatt cactttcagc cattacgcaa tgagctgggt cagacaggca     120

ccaggtaagg gactggagtg ggtctctgca attagcggta gcggtggtag cacttactac     180

gcagacagcg tgaagggtcg cttcaccatc tcacgcgaca actccaagaa caccctgtac     240

ctgcagatga acagccttcg cgcagaggac actgccgtgt attactgcgc acgtggttgg     300

gacagcccta ctctgacata cttcgacagc tggggacaag gtactctggt cactgtctcc     360

tcaggtggag gcggttcagg cggaggtgga tctggcggtg gcggatccca gtctgtgctg     420

actcagccac cttcagcatc tggtactcca ggtcagcgcg tcaccatcag ctgcagcggt     480

actagcagca acatcggtaa caacgacgtg agctggtatc agcaactccc aggcaccgct     540

cctaagctcc tgatttacca ggacactaag cgtcctagcg gtgtgcctga tcgcttttct     600

gggtccaagt ctggcacctc agcctctctg gctatcagtg gacttcgctc cgaggacgag     660

gctgactatt actgcgcagc atgggacgac agcctgagcg gtcctgtgtt cggcggtggg     720

accaaactga ccgtccta                                                   738


<210>    48
<211>    246
<212>    PRT
<213>    Artificial Sequence

<220>
<223>    CB301_OPALTL_B5


<400>    48
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser His Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr

65                  70                  75                  80

Leu Gln Met Asn Ser Leu Arg Ala Glu Asp Thr Ala Val Tyr Tyr Cys
                85                  90                  95

Ala Arg Gly Trp Asp Ser Pro Thr Leu Thr Tyr Phe Asp Ser Trp Gly
            100                 105                 110

Gln Gly Thr Leu Val Thr Val Ser Ser Gly Gly Gly Gly Ser Gly Gly
        115                 120                 125

Gly Gly Ser Gly Gly Gly Gly Ser Gln Ser Val Leu Thr Gln Pro Pro
    130                 135                 140

Ser Ala Ser Gly Thr Pro Gly Gln Arg Val Thr Ile Ser Cys Ser Gly
145                 150                 155                 160

Thr Ser Ser Asn Ile Gly Asn Asn Asp Val Ser Trp Tyr Gln Gln Leu
                165                 170                 175

Pro Gly Thr Ala Pro Lys Leu Leu Ile Tyr Gln Asp Thr Lys Arg Pro
            180                 185                 190

Ser Gly Val Pro Asp Arg Phe Ser Gly Ser Lys Ser Gly Thr Ser Ala
        195                 200                 205

Ser Leu Ala Ile Ser Gly Leu Arg Ser Glu Asp Glu Ala Asp Tyr Tyr
    210                 215                 220

Cys Ala Ala Trp Asp Asp Ser Leu Ser Gly Pro Val Phe Gly Gly Gly
225                 230                 235                 240

Thr Lys Leu Thr Val Leu
                245


<210>    49
<211>    717
<212>    DNA
<213>    Artificial Sequence

<220>
<223>    CB301_OPALTL_E6


<400>    49
gaggtgcagc tgttggagtc tggtggaggc ttggtacagc ctggaggttc tcttcgcctc      60

tcctgtgcag cctccggatt cactttcagc agctacggta tgcattgggt cagacaggca     120

ccaggtaagg gactggagtg ggtctctgca atcagcggta gcggtggtta cacttactac     180

gcagacagcg tgaagggtcg cttcaccatc tcacgcgaca actccaagaa caccctgtac     240

ctgcagatga acagccttcg cgcagaggac actgccgtgt attactgcgc acgctggcat     300

tacagcttcg actactgggg acaaggtact ctggtcactg tctcctcagg tggaggcggt     360

tcaggcggag gtggatctgg cggtggcgga tcccagtctg tgctgactca gccaccttca     420

gcatctggta ctccaggtca gcgcgtcacc atcagctgca gcggtagcag cagcaacatc     480

ggtaacaact acgtgagctg gtatcagcaa ctcccaggca ccgctcctaa gctcctgatt     540

taccgcaaca accagcgccc tagtggtgtg cctgatcgct tttctgggtc caagtctggc 600

acctcagcct ctctggctat cagtggactt cgctccgagg acgaggctga ctattactgc 660

cagagctacg acaacagcaa cgtgctgttc ggcggtggga ccaaactgac cgtccta 717

<210> 50
<211> 239
<212> PRT
<213> Artificial Sequence

<220>
<223> CB301_OPALTL_E6

<400> 50
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ser Ala Ile Ser Gly Ser Gly Gly Tyr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Trp His Tyr Ser Phe Asp Tyr Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Gln Ser Val Leu Thr Gln Pro Pro Ser Ala Ser Gly Thr
    130                 135                 140

Pro Gly Gln Arg Val Thr Ile Ser Cys Ser Gly Ser Ser Ser Asn Ile
145                 150                 155                 160

Gly Asn Asn Tyr Val Ser Trp Tyr Gln Gln Leu Pro Gly Thr Ala Pro
                165                 170                 175

Lys Leu Leu Ile Tyr Arg Asn Asn Gln Arg Pro Ser Gly Val Pro Asp
            180                 185                 190

Arg Phe Ser Gly Ser Lys Ser Gly Thr Ser Ala Ser Leu Ala Ile Ser
        195                 200                 205

Gly Leu Arg Ser Glu Asp Glu Ala Asp Tyr Tyr Cys Gln Ser Tyr Asp
    210                 215                 220

Asn Ser Asn Val Leu Phe Gly Gly Gly Thr Lys Leu Thr Val Leu
225                 230                 235